# EUROPEAN PATENT SPECIFICATION

(11) **EP 1 853 300 B1**
(45) Date of publication and mention of the grant of the patent: **23.07.2014**
(21) Application number: 06735219.5
(22) Date of filing: 16.02.2006
(51) Int. Cl.: A61K 38/17, A61K 31/517, A61P 35/00

(54) **METHODS OF USING DEATH RECEPTOR AGONISTS AND EGFR INHIBITORS**
VERFAHREN ZUR VERWENDUNG VON TODESREZEPTORAGONISTEN UND EGFR-INHIBITOREN
METHODES D'UTILISATION D'AGONISTES DE RECEPTEURS DE MORT ET D'INHIBITEURS D'EGFR

(30) Priority: 18.02.2005 US 61258
(43) Date of publication of application: 14.11.2007
(73) Proprietor: Genentech, Inc., South San Francisco CA 94080-4990 (US)
(72) Inventor: ASHKENAZI, Avi J., San Mateo, California 94402 (US)
(74) Representative: Kiddle, Simon John
(86) International application number: PCT/US2006/005459
(87) International publication number: WO 2006/089015

(56) References cited:
- WO-A-97/25428
- WO-A-2004/101608
- US-A1- 2004 136 950
- US-A1- 2005 020 498
- STEINBACH JOACHIM P ET AL: "CD95-mediated apoptosis of human glioma cells: Modulation by epidermal growth factor receptor activity" BRAIN PATHOLOGY, vol. 12, no. 1, January 2002 (2002-01), pages 12-20, XP009085236 ISSN: 1015-6305
- BURGOS FUSTER L M ET AL: "SELECT CLINICAL TRIALS OF ERLOTINIB (OSI-774) IN NON-SMALL-CELL LUNG CANCER WITH EMPHASIS ON PHASE III OUTCOMES" CLINICAL LUNG CANCER, CANCER INFORMATION GROUP, DALLAS, TX, US, vol. 6, no. SUPPL 1, 2004, pages S24-S29, XP009053756 ISSN: 1525-7304
- "Antitumor activity of the EGFR/TK inhibitor Tarceva@? (erlotinib, OSI-774) in tumor models" EUROPEAN JOURNAL OF CANCER, PERGAMON PRESS, OXFORD, GB, vol. 38, November 2002 (2002-11), pages S63-S64, XP004403644 ISSN: 0959-8049
- HU W ET AL: "Anticancer therapy targeting the apoptotic pathway" LANCET ONCOLOGY, LANCET PUBLISHING GROUP, LONDON, GB, vol. 4, no. 12, December 2003 (2003-12), pages 721-729, XP004809796 ISSN: 1470-2045

### Sequence Listing

<110> Genentech, Inc.
<120> METHODS OF USING DEATH RECEPTOR AGONISTS AND EGFR INHIBITORS
<130> P2101 PCT
<140> US 11/061,258
   <141> 2005-02-18
<160> 6
<210> 1
   <211> 281
   <212> PRT
   <213> Homo sapiens
<400> 1
<210> 2
   <211> 1042
   <212> DNA
   <213> Homo sapiens
<220>
   <221> Unsure
   <222> 447
   <223> Unknown base
<400> 2
   tttcctcact gactataaaa gaatagagaa ggaagggctt cagtgaccgg 50
   ctgcctggct gacttacagc agtcagactc tgacaggatc atggctatga 100
   tggaggtcca ggggggaccc agcctgggac agacctgcgt gctgatcgtg 150
   atcttcacag tgctcctgca gtctctctgt gtggctgtaa cttacgtgta 200
   ctttaccaac gagctgaagc agatgcagga caagtactcc aaaagtggca 250
   ttgcttgttt cttaaaagaa gatgacagtt attgggaccc caatgacgaa 300
   gagagtatga acagcccctg ctggcaagtc aagtggcaac tccgtcagct 350
   cgttagaaag atgattttga gaacctctga ggaaaccatt tctacagttc 400
   aagaaaagca acaaaatatt tctcccctag tgagagaaag aggtccncag 450
   agagtagcag ctcacataac tgggaccaga ggaagaagca acacattgtc 500
   ttctccaaac tccaagaatg aaaaggctct gggccgcaaa ataaactcct 550
   gggaatcatc aaggagtggg cattcattcc tgagcaactt gcacttgagg 600
   aatggtgaac tggtcatcca tgaaaaaggg ttttactaca tctattccca 650
   aacatacttt cgatttcagg aggaaataaa agaaaacaca aagaacgaca 700
   aacaaatggt ccaatatatt tacaaataca caagttatcc tgaccctata 750
   ttgttgatga aaagtgctag aaatagttgt tggtctaaag atgcagaata 800
   tggactctat tccatctatc aagggggaat atttgagctt aaggaaaatg 850
   acagaatttt tgtttctgta acaaatgagc acttgataga catggaccat 900
   gaagccagtt ttttcggggc ctttttagtt ggctaactga cctggaaaga 950
   aaaagcaata acctcaaagt gactattcag ttttcaggat gatacactat 1000
   gaagatgttt caaaaaatct gaccaaaaca aacaaacaga  aa 1042
<210> 3
   <211> 468
   <212> PRT
   <213> Homo sapiens
<400> 3
   Met Ala Pro Pro Pro Ala Arg Val His Leu Gly Ala Phe Leu Ala
   1               5                   10                  15
   Val Thr Pro Asn Pro Gly Ser Ala Ala Ser Gly Thr Glu Ala Ala
                   20                  25                  30
   Ala Ala Thr Pro Ser Lys Val Trp Gly Ser Ser Ala Gly Arg Ile
                   35                  40                  45
   Glu Pro Arg Gly Gly Gly Arg Gly Ala Leu Pro Thr Ser Met Gly
                   50                  55                  60
   Gln His Gly Pro Ser Ala Arg Ala Arg Ala Gly Arg Ala Pro Gly
                   65                  70                  75
   Pro Arg Pro Ala Arg Glu Ala Ser Pro Arg Leu Arg Val His Lys
                   80                  85                  90
   Thr Phe Lys Phe Val Val Val Gly Val Leu Leu Gln Val Val Pro
                   95                  100                 105
   Ser Ser Ala Ala Thr Ile Lys Leu His Asp Gln Ser Ile Gly Thr
                   110                 115                 120
   Gln Gln Trp Glu His Ser Pro Leu Gly Glu Leu Cys Pro Pro Gly
                   125                 130                 135
   Ser His Arg Ser Glu Arg Pro Gly Ala Cys Asn Arg Cys Thr Glu
                   140                 145                 150
   Gly Val Gly Tyr Thr Asn Ala Ser Asn Asn Leu Phe Ala Cys Leu
                   155                 160                 165
   Pro Cys Thr Ala Cys Lys Ser Asp Glu Glu Glu Arg Ser Pro Cys
                   170                 175                 180
   Thr Thr Thr Arg Asn Thr Ala Cys Gln Cys Lys Pro Gly Thr Phe
                   185                 190                 195
   Arg Asn Asp Asn Ser Ala Glu Met Cys Arg Lys Cys Ser Thr Gly
                   200                 205                 210
   Cys Pro Arg Gly Met Val Lys Val Lys Asp Cys Thr Pro Trp Ser
                   215                 220                 225
   Asp Ile Glu Cys Val His Lys Glu Ser Gly Asn Gly His Asn Ile
                   230                 235                 240
   Trp Val Ile Leu Val Val Thr Leu Val Val Pro Leu Leu Leu Val
                   245                 250                 255
   Ala Val Leu Ile Val Cys Cys Cys Ile Gly Ser Gly Cys Gly Gly
                   260                 265                 270
   Asp Pro Lys Cys Met Asp Arg Val Cys Phe Trp Arg Leu Gly Leu
   275 280 285
   Leu Arg Gly Pro Gly Ala Glu Asp Asn Ala His Asn Glu Ile Leu
   290 295 300
   Ser Asn Ala Asp Ser Leu Ser Thr Phe Val Ser Glu Gln Gln Met
   305 310 315
   Glu Ser Gln Glu Pro Ala Asp Leu Thr Gly Val Thr Val Gln Ser
   320 325 330
   Pro Gly Glu Ala Gln Cys Leu Leu Gly Pro Ala Glu Ala Glu Gly
   335 340 345
   Ser Gln Arg Arg Arg Leu Leu Val Pro Ala Asn Gly Ala Asp Pro
   350 355 360
   Thr Glu Thr Leu Met Leu Phe Phe Asp Lys Phe Ala Asn Ile Val
   365 370 375
   Pro Phe Asp Ser Trp Asp Gln Leu Met Arg Gln Leu Asp Leu Thr
   380 385 390
   Lys Asn Glu Ile Asp Val Val Arg Ala Gly Thr Ala Gly Pro Gly
   395 400 405
   Asp Ala Leu Tyr Ala Met Leu Met Lys Trp Val Asn Lys Thr Gly
   410 415 420
   Arg Asn Ala Ser Ile His Thr Leu Leu Asp Ala Leu Glu Arg Met
   425 430 435
   Glu Glu Arg His Ala Lys Glu Lys Ile Gln Asp Leu Leu Val Asp
   440 445 450
   Ser Gly Lys Phe Ile Tyr Leu Glu Asp Gly Thr Gly Ser Ala Val
   455 460 465
   Ser Leu Glu
<210> 4
   <211> 1407
   <212> DNA
   <213> Homo sapiens
<400> 4
   atggcgccac caccagctag agtacatcta ggtgcgttcc tggcagtgac 50
   tccgaatccc gggagcgcag cgagtgggac agaggcagcc gcggccacac 100
   ccagcaaagt gtggggctct tccgcgggga ggattgaacc acgaggcggg 150
   ggccgaggag cgctccctac ctccatggga cagcacggac ccagtgcccg 200
   ggcccgggca gggcgcgccc caggacccag gccggcgcgg gaagccagcc 250
   ctcggctccg ggtccacaag accttcaagt ttgtcgtcgt cggggtcctg 300
   ctgcaggtcg tacctagctc agctgcaacc atgatcaatc aattggcaca 350
   aattggcaca cagcaatggg aacatagccc tttgggagag ttgtgtccac 400
   caggatctca tagatcagaa cgtcctggag cctgtaaccg gtgcacagag 450
   ggtgtgggtt acaccaatgc ttccaacaat ttgtttgctt gcctcccatg 500
   tacagcttgt aaatcagatg aagaagagag aagtccctgc accacgacca 550
   ggaacacagc atgtcagtgc aaaccaggaa ctttccggaa tgacaattct 600
   gctgagatgt gccggaagtg cagcacaggg tgccccagag ggatggtcaa 650
   ggtcaaggat tgtacgccct ggagtgacat cgagtgtgtc cacaaagaat 700
   caggcaatgg acataatata tgggtgattt tggttgtgac tttggttgtt 750
   ccgttgctgt tggtggctgt gctgattgtc tgttgttgca tcggctcagg 800
   ttgtggaggg gaccccaagt gcatggacag ggtgtgtttc tggcgcttgg 850
   gtctcctacg agggcctggg gctgaggaca atgctcacaa cgagattctg 900
   agcaacgcag actcgctgtc cactttcgtc tctgagcagc aaatggaaag 950
   ccaggagccg gcagatttga caggtgtcac tgtacagtcc ccaggggagg 1000
   cacagtgtct gctgggaccg gcagaagctg aagggtctca gaggaggagg 1050
   ctgctggttc cagcaaatgg tgctgacccc actgagactc tgatgctgtt 1100
   ctttgacaag tttgcaaaca tcgtgccctt tgactcctgg gaccagctca 1150
   tgaggcagct ggacctcacg aaaaatgaga tcgatgtggt cagagctggt 1200
   acagcaggcc caggggatgc cttgtatgca atgctgatga aatgggtcaa 1250
   caaaactgga cggaacgcct cgatccacac cctgctggat gccttggaga 1300
   ggatggaaga gagacatgca aaagagaaga ttcaggacct cttggtggac 1350
   tctggaaagt tcatctactt agaagatggc acaggctctg ccgtgtcctt 1400
   ggagtga 1407
<210> 5
   <211> 411
   <212> PRT
   <213> Homo sapiens
<400> 5
<210> 6
   <211> 440
   <212> PRT
   <213> Homo sapiens
<400> 6

## Description

### RELATED APPLICATIONS

### FIELD OF THE INVENTION

The present invention relates to medical uses of Apo2L/TRAIL polypeptide and an EGFR inhibitor for the treatment of cancer where the EGFR inhibitor is is N-(3-ethynylphenyl)-6,7-bis(2-methoxyethoxy)-4-quinazolinamine, or a pharmaceutically acceptable salt or acid thereof.

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, e.g., Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)].

Various molecules, such as tumor necrosis factor-alpha ("TNF-alpha"), tumor necrosis factor-beta ("TNF-beta" or "lymphotoxin-alpha"), lymphotoxin-beta ("LT-beta"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as Apo2L or TRAIL), Apo-3 ligand (also referred to as TWEAK), APRIL, OPG ligand (also referred to as RANK ligand, ODF, or TRANCE), and TALL-1 (also referred to as BlyS, BAFF or THANK) have been identified as members of the tumor necrosis factor ("TNF") family of cytokines [See, e.g., Gruss and Dower, Blood, 85:3378-3404 (1995); Schmid et al., Proc. Natl. Acad. Sci., 83:1881 (1986); Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Chicheportiche et al., Biol. Chem., 272:32401-32410 (1997); Hahne et al., J. Exp. Med., 188:1185-1190 (1998); WO98/28426 published July 2, 1998; WO98/46751 published October 22, 1998; WO/98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)]. Among these molecules, TNF-alpha, TNF-beta, CD30 ligand, 4-1BB ligand, Apo-1 ligand, Apo-2 ligand (Apo2L/TRAIL) and Apo-3 ligand (TWEAK) have been reported to be involved in apoptotic cell death.

Apo2L/TRAIL was identified several years ago as a member of the TNF family of cytokines. [see, e.g., Wiley et al., Immunity, 3:673-682 (1995); Pitti et al., J. Biol. Chem., 271:12687-12690 (1996); US Patent 6,284,236 issued September 4, 2001] The full-length native sequence human Apo2L/TRAIL polypeptide is a 281 amino acid long, Type II transmembrane protein. Some cells can produce a natural soluble form of the polypeptide, through enzymatic cleavage of the polypeptide's extracellular region [Mariani et al., J. Cell. Biol., 137:221-229 (1997)]. Crystallographic studies of soluble forms of Apo2L/TRAIL reveal a homotrimeric structure similar to the structures of TNF and other related proteins [Hymowitz et al., Molec. Cell, 4:563-571 (1999); Hymowitz et al., Biochemistry, 39:633-644 (2000)]. Apo2L/TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. [Hymowitz et al., supra; Bodmer et al., J. Biol. Chem., 275:20632-20637 (2000)]

It has been reported in the literature that Apo2L/TRAIL may play a role in immune system modulation, including autoimmune diseases such as rheumatoid arthritis [see, e.g., Thomas et al., J. Immunol., 161:2195-2200 (1998); Johnsen et al., Cytokine, 11:664-672 (1999); Griffith et al., J. Exp. Med., 189:1343-1353 (1999); Song et al., J. Exp. Med., 191:1095-1103 (2000)].

Soluble forms of Apo2L/TRAIL have also been reported to induce apoptosis in a variety of cancer cells *in vitro,* including colon, lung, breast, prostate, bladder, kidney, ovarian and brain tumors, as well as melanoma, leukemia, and multiple myeloma [see, e.g., Wiley et al., supra; Pitti et al., supra; Rieger et al., FEBS Letters, 427:124-128 (1998); Ashkenazi et al., J. Clin. Invest., 104:155-162 (1999); Walczak et al., Nature Med., 5:157-163 (1999); Keane et al., Cancer Research, 59:734-741 (1999); Mizutani et al., Clin. Cancer Res., 5:2605-2612 (1999); Gazitt, Leukemia, 13:1817-1824 (1999); Yu et al., Cancer Res., 60:2384-2389 (2000); Chinnaiyan et al., Proc. Natl. Acad. Sci., 97:1754-1759 (2000)]. In vivo studies in murine tumor models further suggest that Apo2L/TRAIL, alone or in combination with chemotherapy or radiation therapy, can exert substantial anti-tumor effects [see, e.g., Ashkenazi et al., supra; Walzcak et al., supra; Gliniak et al., Cancer Res., 59:6153-6158 (1999); Chinnaiyan et al., supra; Roth et al., Biochem. Biophys. Res. Comm., 265:1999 (1999)]. In contrast to many types of cancer cells, most normal human cell types appear to be resistant to apoptosis induction by certain recombinant forms of Apo2L/TRAIL [Ashkenazi et al., supra; Walzcak et al., supra]. Jo et al. has reported that a polyhistidine-tagged soluble form of Apo2L/TRAIL induced apoptosis in vitro in normal isolated human, but not non-human, hepatocytes [Jo et al., Nature Med., 6:564-567 (2000); see also, Nagata, Nature Med., 6:502-503 (2000)]. It is believed that certain recombinant Apo2L/TRAIL preparations may vary in terms of biochemical properties and biological activities on diseased versus normal cells, depending, for example, on the presence or absence of a tag molecule, zinc content, and % trimer content [See, Lawrence et al., Nature Med., Letter to the Editor, 7:383-385 (2001); Qin et al., Nature Med., Letter to the Editor, 7:385-386 (2001)].

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Pan et al. have disclosed a TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for Apo2L/TRAIL is described [see also, WO98/51793 published November 19, 1998; WO98/41629 published September 24, 1998]. That molecule is referred to as DR5 (it has also been alternatively referred to as Apo-2; TRAIL-R, TR6, Tango-63, hAPO8, TRICK2 or KILLER [Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/11791 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

A further group of recently identified TNFR family members are referred to as "decoy receptors," which are believed to function as inhibitors, rather than transducers of signaling. This group includes DCR1 (also referred to as TRID, LIT or TRAIL-R3) [Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)] and DCR2 (also called TRUNDD or TRAIL-R4) [Marsters et al., Curr. Biol., 7:1003-1006 (1997); Pan et al., FEBS Letters, 424:41-45 (1998); Degli-Esposti et al., Immunity, 7:813-820 (1997)], both cell surface molecules, as well as OPG [Simonet et al., supra] and DCR3 [Pitti et al., Nature, 396:699-703 (1998)], both of which are secreted, soluble proteins. Apo2L/TRAIL has been reported to bind those receptors referred to as DcR1, DcR2 and OPG.

Apo2L/TRAIL is believed to act through the cell surface "death receptors" DR4 and DR5 to activate caspases, or enzymes that carry out the intracellular cell death program. [See, e.g., Salvesen et al., Cell, 91:443-446 (1997)]. Upon ligand binding, both DR4 and DR5 can trigger apoptosis independently by recruiting and activating the apoptosis initiator, caspase-8, through the death-domain-containing adaptor molecule referred to as FADD/Mort1 [Kischkel et al., Immunity, 12:611-620 (2000); Sprick et al., Immunity, 12:599-609 (2000); Bodmer et al., Nature Cell Biol., 2:241-243 (2000)]. In contrast to DR4 and DR5, the DcR1 and DcR2 receptors do not signal apoptosis.

Certain antibodies which bind to the DR4 and/or DR5 receptors have been reported in the literature. For example, anti-DR4 antibodies directed to the DR4 receptor and having agonistic or apoptotic activity in certain mammalian cells are described in, e.g., WO 99/37684 published July 29, 1999; WO 00/73349 published July 12, 2000; WO 03/066661 published August 14, 2003. See, also, e.g., Griffith et al., J. Immunol., 162:2597-2605 (1999); Chuntharapai et al., J. Immunol., 166:4891-4898 (2001); WO 02/097033 published December 2, 2002; WO 03/042367 published May 22, 2003; WO 03/038043 published May 8, 2003; WO 03/037913 published May 8, 2003. Certain anti-DR5 antibodies have likewise been described, see, e.g., WO 98/51793 published November 8, 1998; Griffith et al., J. Immunol., 162:2597-2605 (1999); Ichikawa et al., Nature Med., 7:954-960 (2001); Hylander et al., "An Antibody to DR5 (TRAIL-Receptor 2) Suppresses the Growth of Patient Derived Gastrointestinal Tumors Grown in SCID mice", Abstract, 2d International Congress on Monoclonal Antibodies in Cancers, Aug. 29-Sept. 1, 2002, Banff, Alberta, Canada; WO 03/038043 published May 8, 2003; WO 03/037913 published May 8, 2003. In addition, certain antibodies having cross-reactivity to both DR4 and DR5 receptors have been described (see, e.g., US patent 6,252,050 issued June 26, 2001).

For a review of the TNF family of cytokines and their receptors, see Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997); Gruss and Dower, supra, and Nagata, Cell, 88:355-365 (1997); Locksley et al., Cell, 104:487-501 (2001); Wallach, "TNF Ligand and TNF/NGF Receptor Families", Cytokine Research, Academic Press, pages 377-411 (2000).

Epidermal Growth Factor Receptor (EGFR) is a member of the type 1 tyrosine kinase family of growth factor receptors, which play critical roles in cellular growth, differentiation, and survival. Activation of these receptors typically occurs via specific ligand binding, resulting in hetero- or homodimerization between receptor family members, with subsequent autophosphorylation of the tyrosine kinase domain. This activation triggers a cascade of intracellular signaling pathways involved in both cellular proliferation (the ras/raf/MAP kinase pathway) and survival (the PI3 kinase/Akt pathway). Members of this family, including EGFR and HER2, have been directly implicated in cellular transformation.

A number of human malignancies are associated with aberrant or overexpression of EGFR and/or overexpression of its specific ligands e.g. transforming growth factor α (Gullick, Br Med Bull 1991, 47:87-98; Modijtahedi and Dean, Int J Oncol 1994, 4:277-96; Salomon et al., Crit Rev Oncol Hematol 1995;19:183-232). EGFR overexpression has been associated with an adverse prognosis in a number of human cancers, including NSCLC. In some instances, overexpression of tumor EGFR has been correlated with both chemoresistance and a poor prognosis (Lei et al., Anticancer Res 1999; 19:221-8; Veale et al., Br J Cancer 1993;68:162-5).

### SUMMARY OF THE INVENTION

The present invention provides in vitro methods of enhancing apoptosis in mammalian cells, comprising contacting said cells with an effective amount of Apo2L/TRAIL polypeptide and the EGFR inhibitor N-(3-ethynylphenyl)-6,7-bis(2-methoxyethoxy)-4-quinazolinamine, or a pharmaceutically acceptable salt or acid thereof

In a further aspect, the present invention provides Apo2L/TRAIL polypeptide and an EGFR inhibitor for use in a method for treating cancer by enhancing apoptosis in one or more mammalian cells, wherein said EGFR inhibitor is N-(3-ethynylphenyl)-6,7-bis(2-methoxyethoxy)-4-quinazolinamine, or a pharmaceutically acceptable salt or acid thereof.

In a further aspect, the present invention provides the use of Apo2L/ TRAIL polypeptide and EGFR inhibitor in the preparation of a medicament for treating cancer by enhancing apoptosis in one or more mammalian cells, wherein said EGFR inhibitor is N-(3-ethynylphenyl)-6,7-bis(2-methoxyethoxy)-4-quinazolinamine, or a pharmaceutically acceptable salt or acid thereof.

### BRIEF DESCRIPTION OF THE DRAWINGS

Figure 1 shows the nucleotide sequence of human Apo-2 ligand cDNA (SEQ ID NO:2) and its derived amino acid sequence (SEQ ID NO:1). The "N" at nucleotide position 447 is used to indicate the nucleotide base may be a "T" or "G".
Figures 2A and 2B show the nucleotide sequence of a cDNA (SEQ ID NO:4) for full length human DR4 and its derived amino acid sequence (SEQ ID NO:3). The respective nucleotide and amino acid sequences for human DR4 are also reported in Pan et al., Science, 276:111 (1997).
Figure 3A shows the 411 amino acid sequence of human DR5 (SEQ ID NO:5) as published in WO 98/51793 on November 19, 1998. A transcriptional splice variant of human DR5 is known in the art. This DR5 splice variant encodes the 440 amino acid sequence of human DR5 (SEQ ID NO:6) shown in Figures 3B and 3C as published in WO 98/35986 on August 20, 1998.
Figure 4 shows respective receptor expression levels (DR4, DR5, EGFR) in H460 cells with or without pre-treatment with Tarceva™ or Taxol^{®}.
Figures 5A-5D illustrate effects of Apo2 ligand and Tarceva™ treatments on various cancer cell lines in vitro.
Figure 6 illustrates effects of Apo2 ligand and Tarceva™ treatments on H460 cancer cells in vivo.

### DETAILED DESCRIPTION OF THE PREFERRED EMBODIMENTS

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

Before the present methods, kits and uses therefor are described, it is to be understood that this invention is not limited to the particular methodology, protocols, cell lines, animal species or genera, constructs, and reagents described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise.

Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

### I. Definitions

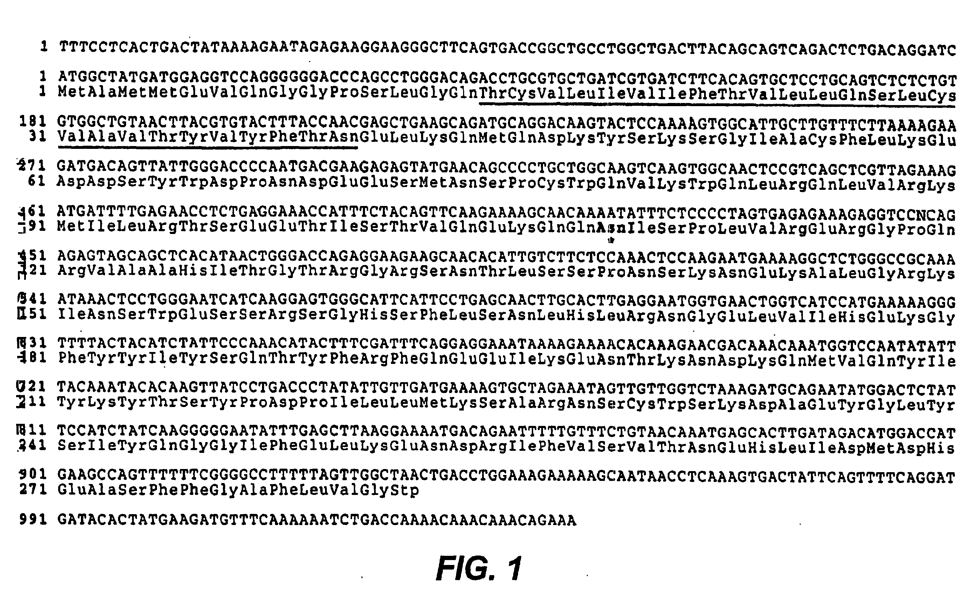

The terms "Apo-2 ligand", "Apo-2L", "Apo2L", "Apo2L/TRAIL", "Apo-2 ligand/TRAIL" and "TRAIL" are used herein interchangeably to refer to a polypeptide sequence which includes amino acid residues 114-281, inclusive, 95-281, inclusive, residues 92-281, inclusive, residues 91-281, inclusive, residues 41-281, inclusive, residues 39-281, inclusive, residues 15-281, inclusive, or residues 1-281, inclusive, of the amino acid sequence shown in Figure 1 (SEQ ID NO:1), as well as biologically active fragments, deletional, insertional, or substitutional variants of the above sequences. In one embodiment, the polypeptide sequence comprises residues 114-281 of Figure 1 (SEQ ID NO:1). Optionally, the polypeptide sequence comprises residues 92-281 or residues 91-281 of Figure 1 (SEQ ID NO:1). The Apo-2L polypeptides may be encoded by the native nucleotide sequence shown in Figure 1 (SEQ ID NO:2). Optionally, the codon which encodes residue Pro119 (Figure 1; SEQ ID NO:2) may be "CCT" or "CCG". Optionally, the fragments or variants are biologically active and have at least about 80% amino acid sequence identity, more preferably at least about 90% sequence identity, and even more preferably, at least 95%, 96%, 97%, 98%, or 99% sequence identity with any one of the above sequences. The definition encompasses substitutional variants of Apo-2 ligand in which at least one of its native amino acids are substituted by another amino acid such as an alanine residue. Optional variants may comprise an amino acid sequence which differs from the native sequence Apo-2 ligand polypeptide sequence of Figure 1 (SEQ ID NO:1) and has one or more of the following amino acid substitutions at the residue position(s) in Figure 1 (SEQ ID NO:1): S96C; S101C; S111C; R170C; K179C. The definition also encompasses a native sequence Apo-2 ligand isolated from an Apo-2 ligand source or prepared by recombinant or synthetic methods. The Apo-2 ligand of the invention includes the polypeptides referred to as Apo-2 ligand or TRAIL disclosed in WO97/01633 published January 16, 1997, WO97/25428 published July 17, 1997, WO99/36535 published July 22, 1999, WO 01/00832 published January 4, 2001, WO02/09755 published February 7, 2002, and WO 00/75191 published December 14, 2000. The terms are used to refer generally to forms of the Apo-2 ligand which include monomer, dimer, trimer, hexamer or hight oligomer forms of the polypeptide. All numbering of amino acid residues referred to in the Apo-2L sequence use the numbering according to Figure 1 (SEQ ID NO:1), unless specifically stated otherwise. For instance, "D203" or "Asp203" refers to the aspartic acid residue at position 203 in the sequence provided in Figure 1 (SEQ ID NO:1).

The term "Apo-2 ligand selective variant" as used herein refers to an Apo-2 ligand polypeptide which includes one or more amino acid mutations in a native Apo-2 ligand sequence and has selective binding affinity for either the DR4 receptor or the DR5 receptor. In one embodiment, the Apo-2 ligand variant has a selective binding affinity for the DR4 receptor and includes one or more amino acid substitutions in any one of positions 189, 191, 193, 199, 201 or 209 of a native Apo-2 ligand sequence. In another embodiment, the Apo-2 ligand variant has a selective binding affinity for the DR5 receptor and includes one or more amino acid substitutions in any one of positions 189, 191, 193, 264, 266, 267 or 269 of a native Apo-2 ligand sequence.

Preferred Apo-2 ligand selective variants include one or more amino acid mutations and exhibit binding affinity to the DR4 receptor which is equal to or greater (≥) than the binding affinity of native sequence Apo-2 ligand to the DR4 receptor, and even more preferably, the Apo-2 ligand variants exhibit less binding affinity (<) to the DR5 receptor than the binding affinity exhibited by native sequence Apo-2 ligand to DR5. When binding affinity of such Apo-2 ligand variant to the DR4 receptor is approximately equal (unchanged) or greater than (increased) as compared to native sequence Apo-2 ligand, and the binding affinity of the Apo-2 ligand variant to the DR5 receptor is less than or nearly eliminated as compared to native sequence Apo-2 ligand, the binding affinity of the Apo-2 ligand variant, for purposes herein, is considered "selective" for the DR4 receptor. Preferred DR4 selective Apo-2 ligand variants of the invention will have at least 10-fold less binding affinity to DR5 receptor (as compared to native sequence Apo-2 ligand), and even more preferably, will have at least 100-fold less binding affinity to DR5 receptor (as compared to native sequence Apo-2 ligand). The respective binding affinity of the Apo-2 ligand variant may be determined and compared to the binding properties of native Apo-2L (such as the 114-281 form) by ELISA, RIA, and/or BIAcore assays, known in the art. Preferred DR4 selective Apo-2 ligand variants of the invention will induce apoptosis in at least one type of mammalian cell (preferably a cancer cell), and such apoptotic activity can be determined by known art methods such as the alamar blue or crystal violet assay. The DR4 selective Apo-2 ligand variants may or may not have altered binding affinities to any of the decoy receptors for Apo-2L, those decoy receptors being referred to in the art as DcR1, DcR2 and OPG.

Further preferred Apo-2 ligand selective variants include one or more amino acid mutations and exhibit binding affinity to the DR5 receptor which is equal to or greater (≥) than the binding affinity of native sequence Apo-2 ligand to the DR5 receptor, and even more preferably, such Apo-2 ligand variants exhibit less binding affinity (<) to the DR4 receptor than the binding affinity exhibited by native sequence Apo-2 ligand to DR4. When binding affinity of such Apo-2 ligand variant to the DR5 receptor is approximately equal (unchanged) or greater than (increased) as compared to native sequence Apo-2 ligand, and the binding affinity of the Apo-2 ligand variant to the DR4 receptor is less than or nearly eliminated as compared to native sequence Apo-2 ligand, the binding affinity of the Apo-2 ligand variant, for purposes herein, is considered "selective" for the DR5 receptor. Preferred DR5 selective Apo-2 ligand variants will have at least 10-fold less binding affinity to DR4 receptor (as compared to native sequence Apo-2 ligand), and even more preferably, will have at least 100-fold less binding affinity to DR4 receptor (as compared to native sequence Apo-2 ligand). The respective binding affinity of the Apo-2 ligand variant may be determined and compared to the binding properties of native Apo2L (such as the 114-281 form) by ELISA, RIA, and/or BIAcore assays, known in the art. Preferred DR5 selective Apo-2 ligand variants will induce apoptosis in at least one type of mammalian cell (preferably a cancer cell), and such apoptotic activity can be determined by known art methods such as the alamar blue or crystal violet assay. The DR5 selective Apo-2 ligand variants may or may not have altered binding affinities to any of the decoy receptors for Apo-2L, those decoy receptors being referred to in the art as DcR1, DcR2 and OPG.

For purposes of shorthand designation of Apo-2 ligand variants described herein, it is noted that numbers refer to the amino acid residue position along the amino acid sequence of the putative native Apo-2 ligand (see Fig. 1).

Amino acid identification herein uses the single-letter alphabet of amino acids, i.e.,

| | | | | | |
|---|---|---|---|---|---|
| Asp | D | Aspartic acid | Ile | I | Isoleucine |
| Thr | T | Threonine | Leu | L | Leucine |
| Ser | S | Serine | Tyr | Y | Tyrosine |
| Glu | E | Glutamic acid | Phe | F | Phenylalanine |
| Pro | P | Proline | His | H | Histidine |
| Gly | G | Glycine | Lys | K | Lysine |
| Ala | A | Alanine | Arg | R | Arginine |
| Cys | C | Cysteine | Trp | W | Tryptophan |
| Val | V | Valine | Gln | Q | Glutamine |
| Met | M | Methionine | Asn | N | Asparagine |

"Death receptor antibody" is used herein to refer generally to antibody or antibodies directed to a receptor in the tumor necrosis factor receptor superfamily and containing a death domain capable of signalling apoptosis, and such antibodies include DR5 antibody and DR4 antibody.

"DR5 receptor antibody", "DR5 antibody", or "anti-DR5 antibody" is used in a broad sense to refer to antibodies that bind to at least one form of a DR5 receptor or extracellular domain thereof. Optionally the DR5 antibody is fused or linked to a heterologous sequence or molecule. Preferably the heterologous sequence allows or assists the antibody to form higher order or oligomeric complexes. Optionally, the DR5 antibody binds to DR5 receptor but does not bind or cross-react with any additional Apo-2L receptor (e.g. DR4, DcR1, or DcR2). Optionally the antibody is an agonist of DR5 signalling activity.

Optionally, the DR5 antibody binds to a DR5 receptor at a concentration range of about 0.1 nM to about 20 mM as measured in a BIAcore binding assay. Optionally, the DR5 antibodies exhibit an Ic 50 value of about 0.6 nM to about 18 mM as measured in a BIAcore binding assay.

"DR4 receptor antibody", "DR4 antibody", or "anti-DR4 antibody" is used in a broad sense to refer to antibodies that bind to at least one form of a DR4 receptor or extracellular domain therof. Optionally the DR4 antibody is fused or linked to a heterologous sequence or molecule. Preferably the heterologous sequence allows or assists the antibody to form higher order or oligomeric complexes. Optionally, the DR4 antibody binds to DR4 receptor but does not bind or cross-react with any additional Apo-2L receptor (e.g. DR5, DcR1, or DcR2). Optionally the antibody is an agonist of DR4 signalling activity.

Optionally, the DR4 antibody binds to a DR4 receptor at a concentration range of about 0.1 nM to about 20 mM as measured in a BIAcore binding assay. Optionally, the DR4 antibodies exhibit an Ic 50 value of about 0.6 nM to about 18 mM as measured in a BIAcore binding assay.

The term "agonist" is used in the broadest sense, and includes any molecule that partially or fully enhances, stimulates or activates one or more biological activities of Apo2L/TRAIL, DR4 or DR5, *in vitro, in situ,* or *in vivo.* Examples of such biological activities binding of Apo2L/TRAIL to DR4 or DR5, include apoptosis as well as those further reported in the literature. An agonist may function in a direct or indirect manner. For instance, the agonist may function to partially or fully enhance, stimulate or activate one or more biological activities of DR4 or DR5, *in vitro, in situ,* or *in vivo* as a result of its direct binding to DR4 or DR5, which causes receptor activation or signal transduction. The agonist may also function indirectly to partially or fully enhance, stimulate or activate one or more biological activities of DR4 or DR5, in vitro, *in* situ, or in vivo as a result of, e.g., stimulating another effector molecule which then causes DR4 or DR5 activation or signal transduction. It is contemplated that an agonist may act as an enhancer molecule which functions indirectly to enhance or increase DR4 or DR5 activation or activity. For instance, the agonist may enhance activity of endogenous Apo-2L in a mammal. This could be accomplished, for example, by pre-complexing DR4 or DR5 or by stabilizing complexes of the respective ligand with the DR4 or DR5 receptor (such as stabilizing native complex formed between Apo-2L and DR4 or DR5).

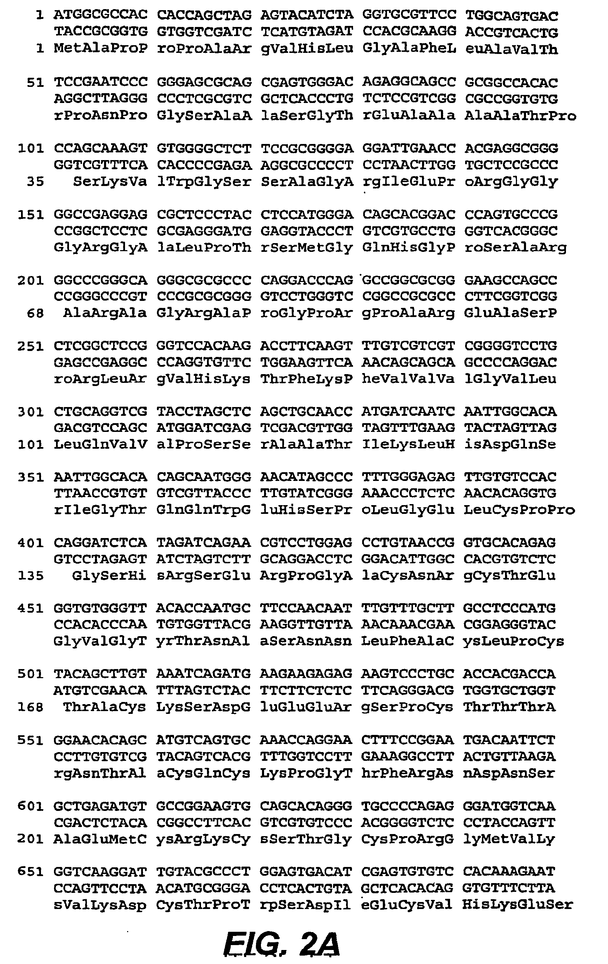

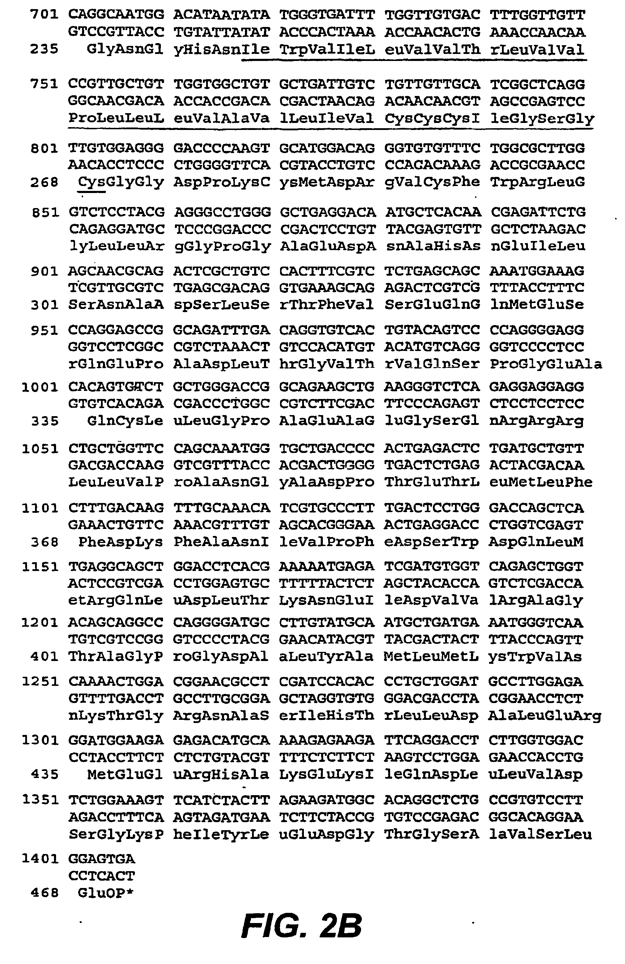

The term "DR4" and "DR4 receptor" as used herein refers to full length and soluble, extracellular domain forms of the receptor described in Pan et al., Science, 276:111-113 (1997); WO98/32856 published July 30, 1998; US Patent 6,342,363 issued January 29, 2002; and WO99/37684 published July 29, 1999. The full length amino acid sequence of DR4 receptor is provided herein in Fig 2.

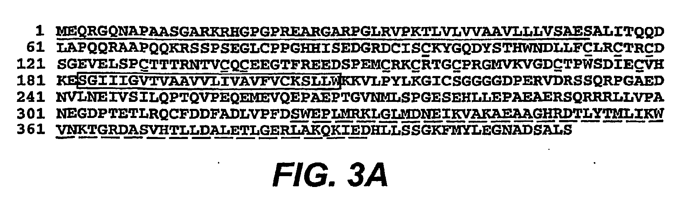

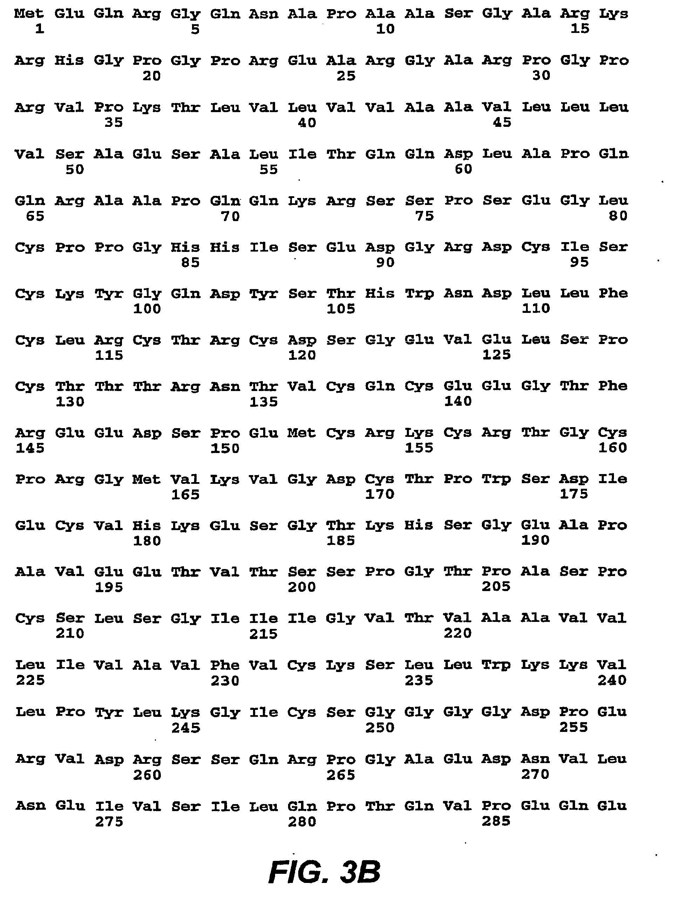

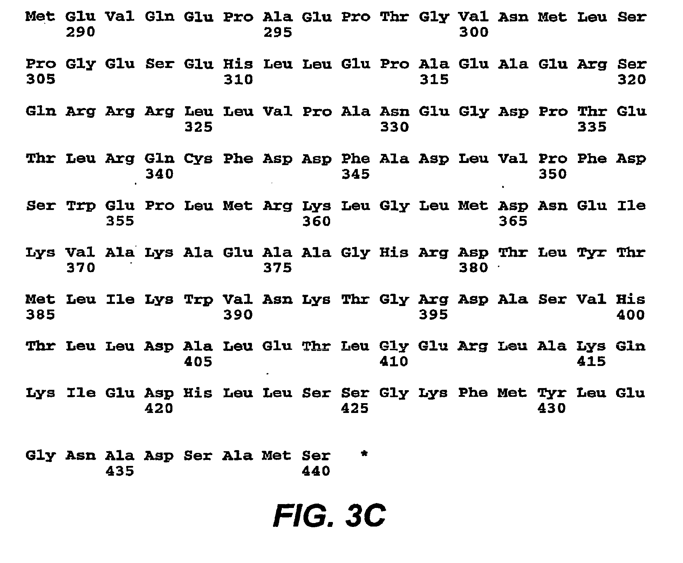

The term "DR5" and "DR5 receptor" as used herein refers to the full length and soluble, extracellular domain forms of the receptor described in Sheridan et al., Science, 277:818-821 (1997); Pan et al., Science, 277:815-818 (1997), US Patent 6,072,047 issued June 6, 2000; US Patent 6,342,369, WO98/51793 published November 19, 1998; WO98/41629 published September 24, 1998; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/11791 published March 11, 1999. The DR5 receptor has also been referred to in the art as Apo-2; TRAIL-R, TR6, Tango-63, hAPO8, TRICK2 or KILLER. The term DR5 receptor used herein includes the full length 411 amino acid polypeptide provided in Fig. 3A and the full length 440 amino acid polypeptide provided in Figs. 3B-C.

The term "polyol" when used herein refers broadly to polyhydric alcohol compounds. Polyols can be any water-soluble poly(alkylene oxide) polymer for example, and can have a linear or branched chain. Preferred polyols include those substituted at one or more hydroxyl positions with a chemical group, such as an alkyl group having between one and four carbons. Typically, the polyol is a poly(alkylene glycol), preferably poly(ethylene glycol) (PEG). However, those skilled in the art recognize that other polyols, such as, for example, poly(propylene glycol) and polyethylene-polypropylene glycol copolymers, can be employed using the techniques for conjugation described herein for PEG. The polyols include those well known in the art and those publicly available, such as from commercially available sources.

The term "conjugate" is used herein according to its broadest definition to mean joined or linked together. Molecules are "conjugated" when they act or operate as if joined.

The term "extracellular domain" or "ECD" refers to a form of ligand or receptor which is essentially free of transmembrane and cytoplasmic domains. Ordinarily, the soluble ECD will have less than 1% of such transmembrane and cytoplasmic domains, and preferably, will have less than 0.5% of such domains.

The term "divalent metal ion" refers to a metal ion having two positive charges. Examples of divalent metal ions for use in the present invention include but are not limited to zinc, cobalt, nickel, cadmium, magnesium, and manganese. Particular forms of such metals that may be employed include salt forms (e.g., pharmaceutically acceptable salt forms), such as chloride, acetate, carbonate, citrate and sulfate forms of the above mentioned divalent metal ions. A preferred divalent metal ion for use in the present invention is zinc, and more preferably, the salt form, zinc sulfate. Divalent metal ions, as described herein, are preferably employed in concentrations or amounts (e.g., effective amounts) which are sufficient to, for example, (1) enhance storage stability of Apo-2L trimers over a desired period of time, (2) enhance production or yield of Apo-2L trimers in a recombinant cell culture or purification method, (3) enhance solubility (or reduce aggregation) of Apo-2L trimers, or (4) enhance Apo-2L trimer formation.

"Isolated," when used to describe the various proteins disclosed herein, means protein that has been identified and separated and/or recovered from a component of its natural environment. Contaminant components of its natural environment are materials that would typically interfere with diagnostic or therapeutic uses for the protein, and may include enzymes, hormones, and other proteinaceous or non-proteinaceous solutes. In preferred embodiments, the protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated protein includes protein in situ within recombinant cells, since at least one component of the Apo-2 ligand natural environment will not be present. Ordinarily, however, isolated protein will be prepared by at least one purification step.

An "isolated" nucleic acid molecule is a nucleic acid molecule that is identified and separated from at least one contaminant nucleic acid molecule with which it is ordinarily associated in the natural source of the nucleic acid. An isolated Apo-2 ligand nucleic acid molecule is other than in the form or setting in which it is found in nature. Isolated Apo-2 ligand nucleic acid molecules therefore are distinguished from the Apo-2 ligand nucleic acid molecule as it exists in natural cells. However, an isolated Apo-2 ligand nucleic acid molecule includes Apo-2 ligand nucleic acid molecules contained in cells that ordinarily express Apo-2 ligand where, for example, the nucleic acid molecule is in a chromosomal location different from that of natural cells.

"Percent (%) amino acid sequence identity" with respect to the sequences identified herein is defined as the percentage of amino acid residues in a candidate sequence that are identical with the amino acid residues in the Apo-2 ligand sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Antibody-dependent cell-mediated cytotoxicity" and "ADCC" refer to a cell-mediated reaction in which nonspecific cytotoxic cells that express Fc receptors (FcRs) *(e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al. PNAS (USA) 95:652-656 (1998).

"Human effector cells" are leukocytes which express one or more FcRs and perform effector functions. Preferably, the cells express at least FcγRIII and carry out ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and Fcγ RIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)). FcRs herein include polymorphisms such as the genetic dimorphism in the gene that encodes FcγRIIIa resulting in either a phenylalanine (F) or a valine (V) at amino acid position 158, located in the region of the receptor that binds to IgG1. The homozygous valine FcγRIIIa (FcγRIIIa-158V) has been shown to have a higher affinity for human IgG1 and mediate increased ADCC *in vitro* relative to homozygous phenylalanine FcγRIIIa (FcγRIIIa-158F) or heterozygous (FcγRIIIa-158F/V) receptors.

"Complement dependent cytotoxicity" or "CDC" refer to the ability of a molecule to lyse a target in the presence of complement. The complement activation pathway is initiated by the binding of the first component of the complement system (C1q) to a molecule *(e.g.* an antibody) complexed with a cognate antigen. To assess complement activation, a CDC assay, e.g. as described in Gazzano-Santoro et al., J. Immunol. Methods 202:163 (1996), may be performed.

The term "antibody" herein is used in the broadest sense and specifically covers intact monoclonal antibodies, polyclonal antibodies, multispecific antibodies *(e.g.* bispecific antibodies) formed from at least two intact antibodies, and antibody fragments so long as they exhibit the desired biological activity.

"Antibody fragments" comprise a portion of an intact antibody, preferably comprising the antigen-binding or variable region thereof. Examples of antibody fragments include Fab, Fab', F(ab')₂, and Fv fragments; diabodies; linear antibodies; single-chain antibody molecules; and multispecific antibodies formed from antibody fragments.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody-dependent cell-mediated cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of antibodies are called α, 5, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plackthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, *e.g.,* U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a non-human primate *(e.g.* Old World Monkey, such as baboon, rhesus or cynomolgus monkey) and human constant region sequences (US Pat No. 5,693,780).

"Humanized" forms of non-human *(e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

The term "hypervariable region" when used herein refers to the amino acid residues of an antibody which are responsible for antigen-binding. The hypervariable region comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" *(e.g.* residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

An antibody "which binds" an antigen of interest, *e.g.* a death receptor such as DR4 or DR5, is one capable of binding that antigen with sufficient affinity and/or avidity such that the antibody is useful as a therapeutic agent for targeting a cell expressing the antigen.

For the purposes herein, "immunotherapy" will refer to a method of treating a mammal (preferably a human patient) with an antibody, wherein the antibody may be an unconjugated or "naked" antibody, or the antibody may be conjugated or fused with heterologous molecule(s) or agent(s), such as one or more cytotoxic agent(s), thereby generating an "immunoconjugate".

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The expression "effective amount" refers to an amount of the combination of a death receptor agonist and an EGFR inhibitor which is effective for preventing, ameliorating or treating the disease or condition in question whether administered simultaneously or sequentially. In particular embodiments, an effective amount is the amount of the death receptor agonist and EGFR inhibitor combination sufficient to enhance, or otherwise increase the propensity (such as synergistically) of a cell to undergo apoptosis, reduce tumour volume, or prolong survival of a mammal having a cancer.

The term "immunosuppressive agent" as used herein for adjunct therapy refers to substances that act to suppress or mask the immune system of the mammal being treated herein. This would include substances that suppress cytokine production, downregulate or suppress self-antigen expression, or mask the MHC antigens. Examples of such agents include 2-amino-6-aryl-5-substituted pyrimidines (see U.S. Pat. No. 4,665,077; nonsteroidal antiinflammatory drugs (NSAIDs); azathioprine; cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e.g*., prednisone, methylprednisolone, dexamethasone, and hydrocortisone; methotrexate (oral or subcutaneous); hydroxycloroquine; sulfasalazine; leflunomide; cytokine or cytokine receptor antagonists including anti-interferon-γ, -β, or -α antibodies, anti-tumor necrosis factor-α antibodies (infliximab or adalimumab), anti-TNFα immunoahesin (etanercept), anti-tumor necrosis factor-β antibodies, anti-interleukin-2 antibodies and anti-IL-2 receptor antibodies; anti-LFA-1 antibodies, including anti-CD11a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90); streptokinase; TGF-β; streptodornase; RNA or DNA from the host; FK506; RS-61443; deoxyspergualin; rapamycin; T-cell receptor (Cohen et al., U.S. Pat. No. 5,114,721); T-cell receptor fragments (Offner et al., Science, 251: 430-432 (1991); WO 90/11294; Ianeway, Nature, 341: 482 (1989); and WO 91/01133); and T cell receptor antibodies (EP 340,109) such as T10B9.

The term "cytotoxic agent" as used herein refers to a substance that inhibits or prevents the function of cells and/or causes destruction of cells. The term is intended to include radioactive isotopes (*e.g*. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, or fragments thereof.

"Synergistic activity" or "synergy" or "synergistic effect" or "synergistic effective amount" for the purposes herein means that the effect observed when employing a combination of Apo2L/TRAIL or death receptor antibody and EGFR inhibitor is (1) greater than the effect achieved when that Apo2L/TRAIL, death receptor antibody or EGFR inhibitor is employed alone (or individually) and (2) greater than the sum added (additive) effect for that Apo2L/TRAIL or death receptor antibody and EGFR inhibitor. Such synergy or synergistic effect can be determined by way of a variety of means known to those in the art. For example, the synergistic effect of Apo2L/TRAIL or death receptor antibody and EGFR inhibitor can be observed in in vitro or in vivo assay formats examining reduction of tumor cell number or tumor mass.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured using well known art methods, for instance, by cell viability assays, FACS analysis or DNA electrophoresis, binding of annexin V, fragmentation of DNA, cell shrinkage, dilation of endoplasmic reticulum, cell fragmentation, and/or formation of membrane vesicles (called apoptotic bodies).

The terms "cancer", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include squamous cell cancer, small-cell lung cancer, non-small cell lung cancer (NSCLC), gastrointestinal cancer, Hodgkin's and non-Hodgkin's lymphoma, pancreatic cancer, glioblastoma, glioma, cervical cancer, ovarian cancer, liver cancer such as hepatic carcinoma and hepatoma, bladder cancer, breast cancer, colon cancer, colorectal cancer, endometrial carcinoma, myeloma (such as multiple myeloma), salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer.

A "B cell malignancy" is a malignancy involving B cells. Examples include Hodgkin's disease, including lymphocyte predominant Hodgkin's disease (LPHD); non-Hodgkin's lymphoma (NHL); follicular center cell (FCC) lymphoma; acute lymphocytic leukemia (ALL); chronic lymphocytic leukemia (CLL); hairy cell leukemia; plasmacytoid lymphocytic lymphoma; mantle cell lymphoma; AIDS or HIV-related lymphoma; multiple myeloma; central nervous system (CNS) lymphoma; post-transplant lymphoproliferative disorder (PTLD); Waldenstrom's macroglobulinemia (lymphoplasmacytic lymphoma); mucosa-associated lymphoid tissue (MALT) lymphoma; and marginal zone lymphoma/leukemia.

Non-Hodgkin's lymphoma (NHL) includes, but is not limited to, low grade/follicular NHL, relapsed or refractory NHL, front line low grade NHL, Stage III/IV NHL, chemotherapy resistant NHL, small lymphocytic (SL) NHL, intermediate grade/follicular NHL, intermediate grade diffuse NHL, diffuse large cell lymphoma, aggressive NHL (including aggressive front-line NHL and aggressive relapsed NHL), NHL relapsing after or refractory to autologous stem cell transplantation, high grade immunoblastic NHL, high grade lymphoblastic NHL, high grade small non-cleaved cell NHL, bulky disease NHL, etc.

The term "prodrug" as used in this application refers to a precursor or derivative form of a pharmaceutically active substance that is less cytotoxic to cancer cells compared to the parent drug and is capable of being enzymatically activated or converted into the more active parent form. See, *e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs also refer to percursors or derivatives of a parent pharmaceutically active substance that has greater bioavailability than the parent substance and is converted to the parent compound in vivo. For example, a prodrug may have greater absorption into the bloodstream upon oral ingestion compared to the parent substance. The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described below.

The term "cytotoxic agent" as used herein refers to a substance that inhibits or prevents the function of cells and/or causes destruction of cells. The term is intended to include radioactive isotopes (*e.g*. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol- Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE^{®} doxetaxel (Rhône-Poulenc Rorer, Antony, France); chloranbucil; GEMZAR^{®} gemcitabine; 6- thioguanine; mercaptopurine; methotrexate; platinum analogs such as cisplatin and carboplatin; vinblastine; platinum; etoposide (VP-16); ifosfamide; mitoxantrone; vincristine; NAVELBINE^{®} vinorelbine; novantrone; teniposide; edatrexate; daunomycin; aminopterin; xeloda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.
Also included in this definition are anti-hormonal agents that act to regulate or inhibit hormone action on tumors such as antiestrogens and selective estrogen receptor modulators (SERMs), including, for example, tamoxifen (including NOLVADEX^{®} tamoxifen), raloxifene, droloxifene, 4-hydroxytamoxifen, trioxifene, keoxifene, LY117018, onapristone, and FARESTON· toremifene; aromatase inhibitors that inhibit the enzyme aromatase, which regulates estrogen production in the adrenal glands, such as, for example, 4(5)-imidazoles, aminoglutethimide, MEGASE^{®} megestrol acetate, AROMASIN^{®} exemestane, formestanie, fadrozole, RIVISOR^{®} vorozole, FEMARA^{®} letrozole, and ARIMIDEX^{®} anastrozole; and antiandrogens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME^{®} ribozyme) and a HER2 expression inhibitor; vaccines such as gene therapy vaccines, for example, ALLOVECTIN^{®} vaccine, LEUVECTIN^{®} vaccine, and VAXID^{®} vaccine; PROLEUKIN^{®} rIL-2; LURTOTECAN^{®} topoisomerase 1 inhibitor; ABARELIX^{®} rmRH; and pharmaceutically acceptable salts, acids or derivatives of any of the above.

A "growth inhibitory agent" when used herein refers to a compound or composition which inhibits growth of a cell, either in vitro or in vivo. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TGF-β; insulin-like growth factor-I and -II; erythropoietin (EPO); osteoinductive factors; interferons such as interferon-α,-β, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12; and other polypeptide factors including LIF and kit ligand (KL). As used herein, the term cytokine includes proteins from natural sources or from recombinant cell culture and biologically active equivalents of the native sequence cytokines.

The terms "treating", "treatment" and "therapy" as used herein refer to curative therapy, prophylactic therapy, and preventative therapy.

The term "mammal" as used herein refers to any mammal classified as a mammal, including humans, cows, horses, dogs and cats. In a preferred embodiment of the invention, the mammal is a human.

The term "EGFR" refers to the receptor tyrosine kinase polypeptide Epidermal Growth Factor Receptor which is described in Ullrich et al, Nature (1984) 309:418425, alternatively referred to as Her-1 and the c-erbB gene product, as well as variants thereof such as EGFRvIII. Variants of EGFR also include deletional, substitutional and insertional variants, for example those described in Lynch et al (New England Journal of Medicine 2004, 350:2129), Paez et al (Science 2004, 304:1497), Pao et al (PNAS 2004, 101:13306).

The term "EGFR inhibitor" refers to compounds that bind to or otherwise interact directly with EGFR and prevent or reduce its signalling activity, and is alternatively referred to as an "EGFR antagonist".

The compounds disclosed herein are members of a class of the EGFR inhibitor having the general formula I: 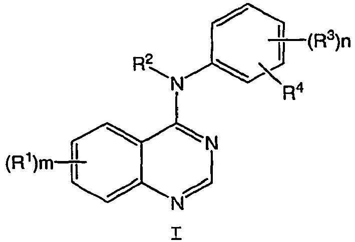 in accordance with US 5,757,498 wherein:
X is halo or hydroxy;
m is 1, 2, or 3;
each R¹ is independently selected from the group consisting of hydrogen, halo, hydroxy, hydroxyamino, carboxy, nitro, guanidino, ureido, cyano, trifluoromethyl, and -(C₁ -C₄ alkylene)-W-(phenyl) wherein W is a single bond, O, S or NH;
or each R¹ is independently selected from R⁹ and C₁-C₄ alkyl substituted by cyano, wherein R⁹ is selected from the group consisting of R⁵, -OR⁶, -NR⁶ R⁶, -C(O)R⁷, -NHOR⁵, -OC(O)R⁶, cyano, A and -YR⁵; R⁵ is C₁-C₄ alkyl; R⁶ is independently hydrogen or R⁵; R⁷ is R⁵, -OR⁶ or -NR⁶R⁶ ; A is selected from piperidino, morpholino, pyrrolidino, 4-R⁶-piperazin-1-yl, imidazol-1-yl, 4-pyridon-1-yl,-(C₁-C₄ alkylene) (CO2H), phenoxy, phenyl, phenylsulfanyl, C₂-C₄ alkenyl, and -(C₁-C₄) alkylene)C(O)NR⁶R⁶; and Y is S, SO, or SO₂; wherein the alkyl moieties in R⁵, -OR⁶ and -NR⁶R⁶ are optionally substituted by one to three halo substituents and the alkyl moieties in R⁵, -OR⁶ and -NR⁶R⁶ are optionally substituted by 1 or 2 R⁹ groups, and wherein the alkyl moieties of said optional substituents are optionally substituted by halo or R⁹, with the proviso that two heteroatoms are not attached to the same carbon atom;
or each R¹ is independently selected from -NHSO₂R⁵, phthalimido-(C₁-C₄)-alkylsulfonylamino, benzamido, benzenesulfonylamino, 3-phenylureido, 2-oxopyrrolidin-1-yl, 2,5-dioxopyrrolidin-1-yl, and R¹⁰-(C₂-C₄)-alkanoylamino wherein R¹⁰ is selected from halo, -OR⁶, C₂-C₄ alkanoyloxy, -C(O)R ⁷, and -NR⁶R⁶; and wherein said -NHSO₂R⁵, phthalimido-(C₁-C₄-alkylsulfonylamino, benzamido, benzenesulfonylamino, 3-phenylureido, 2-oxopyrrolidin-1-yl, 2,5-dioxopyrrolidin-1-yl, and R¹⁰-(C₂-C₄)-alkanoylamino R¹ groups are optionally substituted by 1 or 2 substituents independently selected from halo, C₁-C₄ alkyl, cyano, methanesulfonyl and C₁-C₄ alkoxy;
or two R¹ groups are taken together with the carbons to which they are attached to form a 5-8 membered ring that includes 1 or 2 heteroatoms selected from O, S and N;
R² is hydrogen or C₁-C₆ alkyl optionally substituted by 1 to 3 substituents independently selected from halo, C₁-C₄ alkoxy, -NR⁶R⁶, and -SO₂R⁵;
n is 1 or 2 and each R³ is independently selected from hydrogen, halo, hydroxy, C₁-C₆ alkyl, -NR⁶R⁶, and C₁-C₄ alkoxy, wherein the alkyl moieties of said R³ groups are optionally substituted by 1 to 3 substituents independently selected from halo, C₁-C₄ alkoxy, - NR⁶R⁶, and -SO₂R; and,
R⁴ is azido or - (ethynyl)-R¹¹ wherein R¹¹ is hydrogen or C₁-C₆ alkyl optionally substituted by hydroxy, -OR⁶, or -NR⁶R⁶.

The EGFR inhibitor disclosed herein is a compound according to formula I selected from the group consisting of:
(6,7-dimethoxyquinazolin-4-yl)-(3-ethynylphenyl)-amine; (6,7-dimethoxyquinazolin-4-yl)-[3-(3'-hydroxypropyn-1-yl)phenyl]-amine; [3- (2' - (aminomethyl) -ethynyl) phenyl] - (6, 7-dimethoxyquinazolin-4- yl)-amine; (3-ethynylphenyl)-(6-nitroquinazolin-4-yl)-amine; (6,7-dimethoxyquinazolin-4-yl)-(4-ethynylphenyl)-amine; (6,7-dimethoxyquinazolin-4-yl)-(3-ethynyl-2-methylphenyl)-amine; (6-aminoquinazolin-4-yl)-(3-ethynylphenyl)-amine; (3-ethynylphenyl)-(6-methanesulfonylaminoquinazolin-4-yl)-amine; (3-ethynylphenyl)-(6,7-methylenedioxyquinazolin-4-yl)-amine; (6,7-dimethoxyquinazolin-4-yl)-(3-ethynyl-6-methylphenyl)-amine; (3-ethynylphenyl)-(7-nitroquinazolin-4-yl)-amine; (3-ethynylphenyl)-[6-(4'-toluenesulfonylamino)quinazolin-4-yl]- amine; (3-ethynylphenyl)-{6-[2'-phthalimido-eth-1'-yl-sulfonylamino]quinazolin-4-yl}-amine; (3-ethynylphenyl)-(6-guanidinoquinazolin-4-yl)-amine; (7-aminoquinazolin-4-yl)-(3-ethynylphenyl)-amine; (3-ethynylphenyl)-(7-methoxyquinazolin-4-yl)-amine; (6-carbomethoxyquinazolin-4-yl)-(3-ethynylphenyl)-amine; (7-carbomethoxyquinazolin-4-yl)-(3-ethynylphenyl)-amine; [6,7-bis(2-methoxyethoxy)quinazolin-4-yl]-(3-ethynylphenyl)-amine; (3-azidophenyl)-(6,7-dimethoxyquinazolin-4-yl)-amine; (3-azido-5-chlorophenyl)-(6,7-dimethoxyquinazolin-4-yl)-amine; (4-azidophenyl)-(6,7-dimethoxyquinazolin-4-yl)-amine; (3-ethynylphenyl)-(6-methansulfonyl-quinazolin-4-yl)-amine; (6-ethansulfanyl-quinazolin-4-yl)-(3-ethynylphenyl)-amine; (6,7-dimethoxy-quinazolin-4-yl)-(3-ethynyl-4-fluoro-phenyl)- amine; (6,7-dimethoxy-quinazolin-4-yl)-[3-(propyn-1'-yl)-phenyl]-amine; [6,7-bis-(2-methoxy-ethoxy)-quinazolin-4-yl]-(5-ethynyl-2-methyl-phenyl)-amine; [6,7-bis-(2-methoxy-ethoxy)-quinazolin-4-yl]-(3-ethynyl-4-fluoro- phenyl)-amine; [6,7-bis-(2-chloro-ethoxy)-quinazolin-4-yl]-(3-ethynyl-phenyl)- amine; [6-(2-chloro-ethoxy)-7-(2-methoxy-ethoxy)-quinazolin-4-yl]-(3- ethynyl-phenyl)-amine; [6,7-bis-(2-acetoxy-ethoxy)-quinazolin-4-yl]-(3-ethynyl-phenyl)-amine; 2-[4-(3-ethynyl-phenylamino)-7-(2-hydroxy-ethoxy)-quinazolin-6- yloxy]-ethanol; [6-(2-acetoxy-ethoxy)-7-(2-methoxy-ethoxy)-quinazolin-4-yl]-(3-ethynyl-phenyl)-amine; [7-(2-chloro-ethoxy)-6-(2-methoxy-ethoxy)-quinazolin-4-yl]-(3-ethynyl-phenyl)-amine; [7-(2-acetoxy-ethoxy)-6-(2-methoxy-ethoxy)-quinazolin-4-yl]-(3-ethynyl-phenyl)-amine; 2-[4-(3-ethynyl-phenylamino)-6-(2-hydroxy-ethoxy)-quinazolin-7- yloxy]-ethanol; 2-[4-(3-ethynyl-phenylamino)-7-(2-methoxy-ethoxy)-quinazolin-6-yloxy]-ethanol; 2-[4-(3-ethynyl-phenylamino)-6-(2-methoxy-ethoxy)-quinazolin-7-yloxy]-ethanol; [6-(2-acetoxy-ethoxy)-7-(2-methoxy-ethoxy)-quinazolin-4-yl]-(3-ethynyl-phenyl)-amine; (3-ethynyl-phenyl)-{6-(2-methoxy-ethoxy)-7-[2-(4-methyl-piperazin- 1-yl)-ethoxy]-quinazolin-4-yl}-amine; (3-ethynyl-phenyl)-[7-(2-methoxy-ethoxy)-6-(2-morpholin-4-yl)-ethoxy)-quinazolin-4-yl]-amine; (6,7-diethoxyquinazolin-1-yl)-(3-ethynylphenyl)-amine; (6,7-dibutoxyquinazolin-1-yl)-(3-ethynylphenyl)-amine; (6,7-diisopropoxyquinazolin-1-yl)-(3-ethynylphenyl)-amine; (6,7-diethoxyquinazolin-1-yl)-(3-ethynyl-2-methyl-phenyl)-amine; [6,7-bis-(2-methoxy-ethoxy)-quinazolin-1-yl]-(3-ethynyl-2-methylphenyl)-amine; (3-ethynylphenyl)-[6-(2-hydroxy-ethoxy)-7-(2-methoxy-ethoxy)- quinazolin-1-yl]-amine; [6,7-bis-(2-hydroxy-ethoxy)-quinazolin-1-yl]-(3-ethynylphenyl)- amine; 2-[4-(3-ethynyl-phenylamino)-6-(2-methoxy-ethoxy)-quinazolin-7- yloxy]-ethanol; (6,7-dipropoxy-quinazolin-4-yl)-(3-ethynyl-phenyl)-amine; (6,7-diethoxy-quinazolin-4-yl)-(3-ethynyl-5-fluoro-phenyl)-amine; (6,7-diethoxy-quinazolin-4-yl)-(3-ethynyl-4-fluoro-phenyl)-amine; (6,7-diethoxy-quinazolin-4-yl)-(5-ethynyl-2-methyl-phenyl)-amine; (6,7-diethoxy-quinazolin-4-yl)-(3-ethynyl-4-methyl-phenyl)-amine; (6-aminomethyl-7-methoxy-quinazolin-4-yl)-(3-ethynyl-phenyl)-amine; (6-aminomethyl-7-methoxy-quinazolin-4-yl)-(3-ethynylphenyl)- amine; (6-aminocarbonylmethyl-7-methoxy-quinazolin-4-yl)-(3- ethynylphenyl)-amine; (6-aminocarbonylethyl-7-methoxy-quinazolin-4-yl)-(3- ethynylphenyl)-amine; (6-aminocarbonylmethyl-7-ethoxy-quinazolin-4-yl)-(3- ethynylphenyl)-amine; (6-aminocarbonylethyl-7-ethoxy-quinazolin-4-yl)-(3-ethynylphenyl)- amine; (6-aminocarbonylmethyl-7-isopropoxy-quinazolin-4-yl)-(3- ethynylphenyl)-amine; (6-aminocarbonylmethyl-7-propoxy-quinazolin-4-yl)-(3- ethynylphenyl)-amine; (6-aminocarbonylmethyl-7-methoxy-quinazolin-4-yl)-(3- ethynylphenyl)-amine; (6-aminocarbonylethyl-7-isopropoxy-quinazolin-4-yl)-(3-ethynylphenyl)-amine; and (6-aminocarbonylethyl-7-propoxy-quinazolin-4-yl)-(3- ethynylphenyl)-amine; (6,7-diethoxyquinazolin-1-yl)-(3-ethynylphenyl)-amine; (3-ethynylphenyl)-[6-(2-hydroxy-ethoxy)-7-(2-methoxy-ethoxy)-quinazolin-1-yl]-amine; [6,7-bis-(2-hydroxy-ethoxy)-quinazolin-1-yl]-(3-ethynylphenyl)- amine; [6,7-bis-(2-methoxy-ethoxy)-quinazolin-1-yl]-(3-ethynylphenyl)-amine; (6,7-dimethoxyquinazolin-1-yl)-(3-ethynylphenyl)-amine; (3-ethynylphenyl)-(6-methanesulfonylamino-quinazolin-1-yl)-amine; and (6-amino-quinazolin-1-yl)-(3-ethynylphenyl)-amine.

The EGFR inhibitor of formula I used according to the present invention is N-(3-ethynylphenyl)-6,7-bis(2-methoxyethoxy)-4-quinazolinamine. In a particular embodiment, the EGFR inhibitor N-(3-ethynylphenyl)-6,7-bis(2-methoxyethoxy)-4-quinazolinamine is an HCl salt form. In another particular embodiment, the EGFR inhibitor N-(3-ethynylphenyl)-6,7-bis(2-methoxyethoxy)-4-quinazolinamine is in a substantially homogeneous crystalline polymorph form (described as polymorph B in WO 01/34,574) that exhibits an X-ray powder diffraction pattern having characteristic peaks expressed in degrees 2-theta at approximately 6.26, 12.48, 13.39, 16.96, 20.20, 21.10, 22.98, 24.46, 25.14 and 26.91. Such polymorph form of N-(3-ethynylphenyl)-6,7-bis(2-methoxyethoxy)-4-quinazolinamine is referred to as Tarceva™ as well as OSI-774, CP-358774 and erlotinib.

The compounds of formula I, pharmaceutically acceptable salts and prodrugs thereof (hereafter the active compounds) may be prepared by any process known to be applicable to the preparation of chemically-related compounds. In general the active compounds may be made from the appropriately substituted quinazoline using the appropriately substituted amine as shown in the general scheme I disclosed in US 5,747,498:

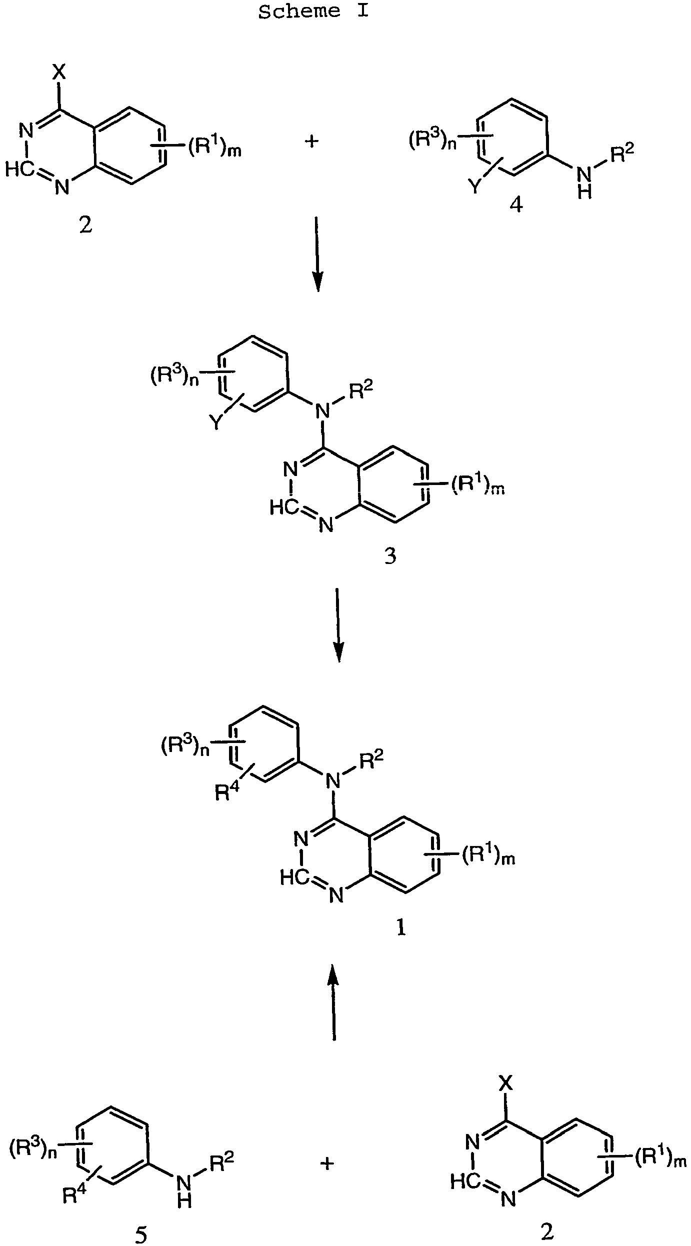

As shown in Scheme I the appropriate 4-substituted quinazoline 2 wherein x is a suitable displaceable leaving group such as halo, aryloxy, alkylsulfinyl, alkylsulfonyl such as trifluoromethanesulfonyloxy, arylsulfinyl, arylsulfonyl, siloxy, cyano, pyrazolo, triazolo or tetrazolo, preferably a 4-chloroquinazoline, is reacted with the appropriate amine or amine hydrochloride 4 or 5, wherein R⁴ is as described above and Y is Br, I, or trifluoromethane-sulfonyloxy in a solvent such as a (C₁-C₆)alcohol, dimethylformamide (DMF), N-methylpyrrolidin-2-one, chloroform, acetonitrile, tetrahydrofuran (THF), 1-4 dioxane, pyridine or other aprotic solvent. The reaction may be effected in the presence of a base, preferably an alkali or alkaline earth metal carbonate or hydroxide or a tertiary amine base, such as pyridine, 2,6-lutidine, collidine, N- methyl- morpholine, triethylamine, 4-dimethylamino-pyridine or N,N-dimethylaniline. These bases are hereinafter refered to as suitable bases. The reaction mixture is maintained at a temperature from about ambient to about the reflux temperature of the solvent, preferably from about 35°C to about reflux, until substantially no remaining 4-haloquinazoline can be detected, typically about 2 to about 24 hours. Preferably, the reaction is performed under an inert atmosphere such as dry nitrogen.

Generally the reactants are combined stoichiometrically. When an amine base is used for those compounds where a salt (typically the HCl salt) of an amine 4 or 5 is used, it is preferable to use excess amine base, generally an extra equivalent of amine base. (Alternatively, if an amine base is not used an excess of the amine 4 or 5 may be used).

For those compounds where a sterically hindered amine 4 (such as a 2-alkyl-3-ethynylaniline) or very reactive 4-haloquinazoline is used it is preferable to use t-butyl alcohol or a polar aprotic solvent such as DMF or N-methylpyrrolidin-2-one as the solvent.

Alternatively, a 4-substituted quinazoline 2 wherein X is hydroxyl or oxo (and the 2-nitrogen is hydrogenated) is reacted with carbon tetrachloride and an optionally substituted triarylphosphine which is optionally supported on an inert polymer (e.g. triphenylphosphine, polymer supported, Aldrich Cat. No. 36,645-5, which is a 2% divinylbenzene cross-linked polystyrene containing 3 mmol phosphorous per gram resin) in a solvent such as carbon tetrachloride, chloroform, dichloroethane, tetrahydrofuran, acetonitrile or other aprotic solvent or mixtures thereof. The reaction mixture is maintained at a temperature from about ambient to reflux, preferably from about 35°C to reflux, for 2 to 24 hours. This mixture is reacted with the appropriate amine or amine hydrochloride 4 or 5 either directly or after removal of solvent, for example by vacuum evaporation, and addition of a suitable alternative solvent such as a (C₁-C₆) alcohol, DMF, N-methylpyrrolidin-2-one, pyridine or 1-4 dioxane. Then, the reaction mixture is maintained at a temperature from about ambient to the reflux temperature of the solvent preferably from about 35°C to about reflux, until substantially complete formation of product is acheived, typically from about 2 to about 24 hours. Preferably the reaction is performed under an inert atmosphere such as dry nitrogen.

When compound 4, wherein Y is Br, I, or trifluoromethanesulfonyloxy, is used as starting material in the reaction with quinazoline 2, a compound of formula 3 is formed wherein R¹, R², R³, and Y are as described above. Compound 3 is converted to compounds of formula 1 wherein R⁴ is R¹¹ ethynyl, and R¹¹ is as defined above, by reaction with a suitable palladium reagent such as tetrakis(triphenylphosphine)palladium or bis(triphenylphosphine)palladium dichloride in the presence of a suitable Lewis acid such as cuprous chloride and a suitable alkyne such as trimethylsilylacetylene, propargyl alcohol or 3-(N,N-dimethylamino)-propyne in a solvent such as diethylamine or triethylamine. Compounds 3, wherein Y is NH₂, may be converted to compounds 1 wherein R⁴ is azide by treatment of compound 3 with a diazotizing agent, such as an acid and a nitrite (e.g., acetic acid and NaNO₂) followed by treatment of the resulting product with an azide, such as NaN₃.

For the production of those compounds of Formula I wherein an R¹ is an amino or hydroxyamino group the reduction of the corresponding Formula I compound wherein R¹ is nitro is employed.

The reduction may conveniently be carried out by any of the many procedures known for such transformations. The reduction may be carried out, for example, by hydrogenation of the nitro compound in a reaction-inert solvent in the presence of a suitable metal catalyst such as palladium, platinum or nickel. A further suitable reducing agent is, for example, an activated metal such as activated iron (produced by washing iron powder with a dilute solution of an acid such as hydrochloric acid). Thus, for example, the reduction may be carried out by heating a mixture of the nitro compound and the activated metal with concentrated hydrochloric acid in a solvent such as a mixture of water and an alcohol, for example, methanol or ethanol, to a temperature in the range, for example, 50° to 150° C., conveniently at or near 70°C. Another suitable class of reducing agents are the alkali metal dithionites, such as sodium dithionite, which may be used in (C₁-C₄)alkanoic acids, (C₁-C₆)alkanols, water or mixtures thereof.

For the production of those compounds of Formula I wherein R² or R³ incorporates a primary or secondary amino moiety (other than the amino group intended to react with the quinazoline), such free amino group is preferably protected prior to the above described reaction followed by deprotection, subsequent to the above described reaction with 4-(substituted)quinazoline 2.

Several well known nitrogen protecting groups can be used. Such groups include (C₁-C₆)alkoxycarbonyl, optionally substituted benzyloxycarbonyl, aryloxycarbonyl, trityl, vinyloxycarbonyl, O-nitrophenylsulfonyl, diphenylphosphinyl, p-toluenesulfonyl, and benzyl. The addition of the nitrogen protecting group may be carried out in a chlorinated hydrocarbon solvent such as methylene chloride or 1,2-dichloroethane, or an ethereal solvent such as glyme, diglyme or THF, in the presence or absence of a tertiary amine base such as triethylamine, diisopropylethylamine or pyridine, preferably triethylamine, at a temperature from about 0°C to about 50°C, preferably about ambient temperature. Alternatively, the protecting groups are conveniently attached using Schotten-Baumann conditions.

Subsequent to the above described coupling reaction, of compounds 2 and 5, the protecting group may be removed by deprotecting methods known to those skilled in the art such as treatment with trifluoroacetic acid in methylene chloride for the tert- butoxycarbonyl protected products.

For a description of protecting groups and their use, see T. W. Greene and P. G. M. Wuts, "Protective Groups in Organic Synthesis" Second Ed., John Wiley & Sons, New York, 1991.

For the production of compounds of Formula I wherein R¹ or R² is hydroxy, cleavage of a Formula I compound wherein R¹ or R² is (C₁-C₄)alkoxy is preferred.

The cleavage reaction may conveniently be carried out by any of the many procedures known for such a transformation. Treatment of the protected formula I derivative with molten pyridine hydrochloride (20-30 eq.) at 150° to 175°C may be employed for O-dealkylations. Alternatively, the cleavage reaction may be carried out, for example, by treatment of the protected quinazoline derivative with an alkali metal (C₁-C₄)alkylsulphide, such as sodium ethanethiolate or by treatment with an alkali metal diarylphosphide such as lithium diphenylphosphide. The cleavage reaction may also, conveniently, be carried out by treatment of the protected quinazoline derivative with a boron or aluminum trihalide such as boron tribromide. Such reactions are preferably carried out in the presence of a reaction- inert solvent at a suitable temperature.

Compounds of formula I, wherein R¹ or R² is a (C₁-C₄)alkylsulphinyl or (C₁-C₄)alkylsulphonyl group are preferably prepared by oxidation of a formula I compound wherein R¹ or R² is a (C₁-C₄)alkylsulfanyl group. Suitable oxidizing agents are known in the art for the oxidation of sulfanyl to sulphinyl and/or sulphonyl, e.g., hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible using the stoichiometric amount of oxidizing agent in order to reduce the risk of over oxidation and damage to other functional groups. In general, the reaction is carried out in a suitable solvent such as methylene chloride, chloroform, acetone, tetrahydrofuran or tert-butyl methyl ether and at a temperature from about -25° to 50°C, preferably at or near ambient temperature, i.e., in the range of 15° to 35°C. When a compound carrying a sulphinyl group is desired a milder oxidizing agents should be used such as sodium or potassium metaperiodate, conveniently in a polar solvent such as acetic acid or ethanol. The compounds of formula I containing a (C₁-C₄)alkylsulphonyl group may be obtained by oxidation of the corresponding (C₁-C₄)alkylsulphinyl compound as well as of the corresponding (C₁-C₄)alkylsulfanyl compound.

Compounds of formula I wherein R¹ is optionally substituted (C₂-C₄)alkanoylamino, ureido, 3-phenylureido, benzamido or sulfonamido can be prepared by acylation or sulfonylation of a corresponding compound wherein R¹ is amino. Suitable acylating agents are any agents known in the art for the acylation of amino to acylamino, for example, acyl halides, e.g., a (C₂-C₄)alkanoyl chloride or bromide or a benzoyl chloride or bromide, alkanoic acid anhydrides or mixed anhydrides (e.g., acetic anhydride or the mixed anhydride formed by the reaction of an alkanoic acid and a (C₁-C₄)alkoxycarbonyl halide, for example (C₁-C₄)alkoxycarbonyl chloride, in the presence of a suitable base. For the production of those compounds of Formula I wherein R¹ is ureido or 3-phenylureido, a suitable acylating agent is, for example, a cyanate, e.g., an alkali metal cyanate such as sodium cyanate, or an isocyanate such as phenyl isocyanate. N-sulfonylations may be carried out with suitable sulfonyl halides or sulfonylanhydrides in the presence of a tertiary amine base. In general the acylation or sulfonylation is carried out in a reaction-inert solvent and at a temperature in the range of about -30° to 120°C, conveniently at or near ambient temperature.

Compounds of Formula I wherein R¹ is (C₁-C₄)alkoxy or substituted (C₁-C₄)alkoxy or R¹ is (C₁-C₄)alkylamino or substituted mono-N- or di-N,N-(C₁-C₄)alkylamino, are prepared by the alkylation, preferably in the presence of a suitable base, of a corresponding compound wherein R¹ is hydroxy or amino, respectively. Suitable alkylating agents include alkyl or substituted alkyl halides, for example, an optionally substituted (C₁-C₄)alkyl chloride, bromide or iodide, in the presence of a suitable base in a reaction-inert solvent and at a temperature in the range of about 10° to 140°C, conveniently at or near ambient temperature.

For the production of those compounds of Formula I wherein R¹ is an amino-, oxy- or cyano-substituted (C₁-C₄)alkyl substituent, a corresponding compound wherein R¹ is a (C₁-C₄)alkyl substituent bearing a group which is displacable by an amino-, alkoxy-, or cyano group is reacted with an appropriate amine, alcohol or cyanide, preferably in the presence of a suitable base. The reaction is preferably carried out in a reaction-inert solvent or diluent and at a temperature in the range of about 10° to 100°C, preferably at or near ambient temperature.

Compounds of Formula I, wherein R¹ is a carboxy substituent or a substituent which includes a carboxy group are prepared by hydrolysis of a corresponding compound wherein R¹ is a (C₁-C₄)alkoxycarbonyl substituent or a substituent which includes a (C₁-C₄)alkoxycarbonyl group. The hydrolysis may conveniently be performed, for example, under basic conditions, e.g., in the presence of alkali metal hydroxide.

Compounds of Formula I wherein R¹ is amino, (C₁-C₄)alkylamino, di-[(C₁-C₄)alkyl]amino, pyrrolidin-1-yl, piperidino, morpholino, piperazin-1-yl, 4-(C₁-C₄)alkylpiperazin-1-yl or (C₁-C₉)alkysulfanyl, may be prepared by the reaction, in the presence of a suitable base, of a corresponding compound wherein R¹ is an amine or thiol displaceable group with an appropriate amine or thiol. The reaction is preferably carried out in a reaction-inert solvent or diluent and at a temperature in the range of about 10° to 180°C, conveniently in the range 100° to 150°C.

Compounds of Formula I wherein R¹ is 2-oxopyrrolidin-1-yl or 2-oxopiperidin-1-yl are prepared by the cyclisation, in the presence of a suitable base, of a corresponding compound wherein R¹ is a halo-(C₂-C₄)alkanoylamino group. The reaction is preferably carried out in a reaction-inert solvent or diluent and at a temperature in the range of about 10° to 100°C, conveniently at or near ambient temperature.

For the production of compounds of Formula I in which R¹ is carbamoyl, substituted carbamoyl, alkanoyloxy or substituted alkanoyloxy, the carbamoylation or acylation of a corresponding compound wherein R¹ is hydroxy is convenient.

Suitable acylating agents known in the art for acylation of hydroxyaryl moieties to alkanoyloxyaryl groups include, for example, (C₂-C₄)alkanoyl halides, (C₂-C₄)alkanoyl anhydrides and mixed anhydrides as described above, and suitable substituted derivatives thereof may be employed, typically in the presence of a suitable base. Alternatively, (C₂-C₄)alkanoic acids or suitably substituted derivatives thereof may be coupled with a Formula I compound wherein R¹ is hydroxy with the aid of a condensing agent such as a carbodiimide. For the production of those compounds of Formula I in which R¹ is carbamoyl or substituted carbamoyl, suitable carbamoylating agents are, for example, cyanates or alkyl or arylisocyanates, typically in the presence of a suitable base. Alternatively, suitable intermediates such as the chloroformate or carbonylimidazolyl derivative of a compound of Formula I in which R¹ is hydroxy may be generated, for example, by treatment of said derivative with phosgene (or a phosgene equivalent) or carbonyidiimidazole. The resulting intermediate may then be reacted with an appropriate amine or substituted amine to produce the desired carbamoyl derivatives.

Compounds of formula I wherein R¹ is aminocarbonyl or a substituted aminocarbonyl can be prepared by the aminolysis of a suitable intermediate in which R¹ is carboxy.

The activation and coupling of formula I compounds wherein R¹ is carboxy may be performed by a variety of methods known to those skilled in the art. Suitable methods include activation of the carboxyl as an acid halide, azide, symmetric or mixed anhydride, or active ester of appropriate reactivity for coupling with the desired amine. Examples of such types of intermediates and their production and use in couplings with amines may be found extensively in the literature; for example M. Bodansky and A. Bodansky, "The Practice of Peptide Synthesis", Springer-Verlag, New York, 1984. The resulting formula I compounds may be isolated and purified by standard methods, such as solvent removal and recrystallization or chromatography.

The starting materials for the described reaction scheme I (e.g., amines, quinazolines and amine protecting groups) are readily available or can be easily synthesized by those skilled in the art using conventional methods of organic synthesis. For example, the preparation of 2,3-dihydro-1,4-benzoxazine derivatives are described in R. C. Elderfield, W. H. Todd, S. Gerber, Ch. 12 in "Heterocyclic Compounds", Vol. 6, R. C. Elderfield ed., John Wiley and Sons, Inc., N.Y., 1957. Substituted 2,3-dihydrobenzothiazinyl compounds are described by R. C. Elderfield and E. E. Harris in Ch. 13 of Volume 6 of the Elderfield "Heterocyclic Compounds" book.

### II. Compositions and Methods of the Invention

A cytokine related to the TNF ligand family, the cytokine identified herein as "Apo-2 ligand" or "TRAIL" has been described. The predicted mature amino acid sequence of native human Apo-2 ligand contains 281 amino acids, and has a calculated molecular weight of approximately 32.5 kDa. The absence of a signal sequence and the presence of an internal hydrophobic region suggest that Apo-2 ligand is a type II transmembrane protein. Soluble extracellular domain Apo-2 ligand polypeptides have also been described. See, e.g., WO97/25428 published July 17, 1997. Apo-2L substitutional variants have further been described. Alanine scanning techniques have been utilized to identify various substitutional variant molecules having biological activity. Particular substitutional variants of the Apo-2 ligand include those in which at least one amino acid is substituted by another amino acids such as an alanine residue. These substitutional variants are identified, for example, as "D203A"; "D218A" and "D269A." This nomenclature is used to identify Apo-2 ligand variants wherein the aspartic acid residues at positions 203, 218, and/or 269 (using the numbering shown in Figure 1) are substituted by alanine residues. Optionally, the Apo-2L variants of the present invention may comprise one or more of the amino acid substitutions. Optionally, such Apo-2L variants will be DR4 or DR5 receptor selective variants.

The description below relates to methods of producing Apo-2 ligand, including Apo-2 ligand variants, by culturing host cells transformed or transfected with a vector containing Apo-2 ligand encoding nucleic acid and recovering the polypeptide from the cell culture.

The DNA encoding Apo-2 ligand may be obtained from any cDNA library prepared from tissue believed to possess the Apo-2 ligand mRNA and to express it at a detectable level. Accordingly, human Apo-2 ligand DNA can be conveniently obtained from a cDNA library prepared from human tissues, such as the bacteriophage library of human placental cDNA as described in WO97/25428. The Apo-2 ligand-encoding gene may also be obtained from a genomic library or by oligonucleotide synthesis.

Libraries can be screened with probes (such as antibodies to the Apo-2 ligand or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding Apo-2 ligand is to use PCR methodology [Sambrook et al., supra; Dieffenbach et al., PCR Primer:A Laboratory Manual (Cold Spring Harbor Laboratory Press, 1995)].

Amino acid sequence fragments or variants of Apo-2 ligand can be prepared by introducing appropriate nucleotide changes into the Apo-2 ligand DNA, or by synthesis of the desired Apo-2 ligand polypeptide. Such fragments or variants represent insertions, substitutions, and/or deletions of residues within or at one or both of the ends of the intracellular region, the transmembrane region, or the extracellular region, or of the amino acid sequence shown for the full-length Apo-2 ligand in Figure 1. Any combination of insertion, substitution, and/or deletion can be made to arrive at the final construct, provided that the final construct possesses, for instance, a desired biological activity, such as apoptotic activity, as defined herein. In a preferred embodiment, the fragments or variants have at least about 80% amino acid sequence identity, more preferably, at least about 90% sequence identity, and even more preferably, at least 95%, 96%, 97%, 98% or 99% sequence identity with the sequences identified herein for the intracellular, transmembrane, or extracellular domains of Apo-2 ligand, or the full-length sequence for Apo-2 ligand. The amino acid changes also may alter post-translational processes of the Apo-2 ligand, such as changing the number or position of glycosylation sites or altering the membrane anchoring characteristics.

Variations in the Apo-2 ligand sequence as described above can be made using any of the techniques and guidelines for conservative and non-conservative mutations set forth in U.S. Pat. No. 5,364,934. These include oligonucleotide-mediated (site-directed) mutagenesis, alanine scanning, and PCR mutagenesis.

Scanning amino acid analysis can be employed to identify one or more amino acids along a contiguous sequence. Among the preferred scanning amino acids are relatively small, neutral amino acids. Such amino acids include alanine, glycine, serine and cysteine. Alanine is typically a preferred scanning amino acid among this group because it eliminates the side-chain beyond the beta-carbon and is less likely to alter the main-chain conformation of the variant. [Cunningham et al., Science, 244:1081 (1989)]. Alanine is also typically preferred because it is the most common amino acid. Further, it is frequently found in both buried and exposed positions [Creighton, The Proteins, (W.H. Freeman & Co., NY); Chothia, J. Mol. Biol., 150:1 (1976)].

Amino acids may be grouped according to similarities in the properties of their side chains (in A. L. Lehninger, in Biochemistry, second ed., pp. 73-75, Worth Publishers, New York (1975)):
(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Variations in the Apo-2 ligand sequence also included within the scope of the invention relate to amino-terminal derivatives or modified forms. Such Apo-2 ligand sequences include any of the Apo-2 ligand polypeptides described herein having a methionine or modified methionine (such as formyl methionyl or other blocked methionyl species) at the N-terminus of the polypeptide sequence.

The nucleic acid (e.g., cDNA or genomic DNA) encoding native or variant Apo-2 ligand may be inserted into a replicable vector for further cloning (amplification of the DNA) or for expression. Various vectors are publicly available. The vector components generally include, but are not limited to, one or more of the following: a signal sequence, an origin of replication, one or more marker genes, an enhancer element, a promoter, and a transcription termination sequence, each of which is described below. Optional signal sequences, origins of replication, marker genes, enhancer elements and transcription terminator sequences that may be employed are known in the art and described in further detail in WO97/25428.

Expression and cloning vectors usually contain a promoter that is recognized by the host organism and is operably linked to the Apo-2 ligand nucleic acid sequence. Promoters are untranslated sequences located upstream (5') to the start codon of a structural gene (generally within about 100 to 1000 bp) that control the transcription and translation of a particular nucleic acid sequence, such as the Apo-2 ligand nucleic acid sequence, to which they are operably linked. Such promoters typically fall into two classes, inducible and constitutive. Inducible promoters are promoters that initiate increased levels of transcription from DNA under their control in response to some change in culture conditions, e.g., the presence or absence of a nutrient or a change in temperature. At this time a large number of promoters recognized by a variety of potential host cells are well known. These promoters are operably linked to Apo-2 ligand encoding DNA by removing the promoter from the source DNA by restriction enzyme digestion and inserting the isolated promoter sequence into the vector. Both the native Apo-2 ligand promoter sequence and many heterologous promoters may be used to direct amplification and/or expression of the Apo-2 ligand DNA.

Promoters suitable for use with prokaryotic and eukaryotic hosts are known in the art, and are described in further detail in WO97/25428.

A preferred method for the production of soluble Apo-2L in E. coli employs an inducible promoter for the regulation of product expression. The use of a controllable, inducible promoter allows for culture growth to the desirable cell density before induction of product expression and accumulation of significant amounts of product which may not be well tolerated by the host.

Several inducible promoter systems (T7 polymerase, trp and alkaline phosphatase (AP)) have been evaluated by Applicants for the expression of Apo-2L (form 114-281). The use of each of these three promoters resulted in significant amounts of soluble, biologically active Apo-2L trimer being recovered from the harvested cell paste. The AP promoter is preferred among these three inducible promoter systems tested because of tighter promoter control and the higher cell density and titers reached in harvested cell paste.

Construction of suitable vectors containing one or more of the above-listed components employs standard ligation techniques. Isolated plasmids or DNA fragments are cleaved, tailored, and religated in the form desired to generate the plasmids required.

For analysis to confirm correct sequences in plasmids constructed, the ligation mixtures can be used to transform E. *coli* K12 strain 294 (ATCC 31,446) and successful transformants selected by ampicillin or tetracycline resistance where appropriate. Plasmids from the transformants are prepared, analyzed by restriction endonuclease digestion, and/or sequenced using standard techniques known in the art. [See, e.g., Messing et al., Nucleic Acids Res., 9:309 (1981); Maxam et al., Methods in Enzymology, 65:499 (1980)].

Expression vectors that provide for the transient expression in mammalian cells of DNA encoding Apo-2 ligand may be employed. In general, transient expression involves the use of an expression vector that is able to replicate efficiently in a host cell, such that the host cell accumulates many copies of the expression vector and, in turn, synthesizes high levels of a desired polypeptide encoded by the expression vector [Sambrook et al., supra]. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of Apo-2 ligand that are biologically active Apo-2 ligand.

Other methods, vectors, and host cells suitable for adaptation to the synthesis of Apo-2 ligand in recombinant vertebrate cell culture are described in Gething et al., Nature, 293:620-625 (1981); Mantei et al., Nature, 281:40-46 (1979); EP 117,060; and EP 117,058.

Suitable host cells for cloning or expressing the DNA in the vectors herein include prokaryote, yeast, or higher eukaryote cells. Suitable prokaryotes for this purpose include but are not limited to eubacteria, such as Gram-negative or Gram-positive organisms, for example, Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* Preferably, the host cell should secrete minimal amounts of proteolytic enzymes.

In addition to prokaryotes, eukaryotic microbes such as filamentous fungi or yeast are suitable cloning or expression hosts for Apo-2 ligand-encoding vectors. Suitable host cells for the expression of glycosylated Apo-2 ligand are derived from multicellular organisms. Examples of all such host cells, including CHO cells, are described further in WO97/25428.

Host cells are transfected and preferably transformed with the above-described expression or cloning vectors for Apo-2 ligand production and cultured in nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in Sambrook et al., supra, or electroporation is generally used for prokaryotes or other cells that contain substantial cell-wall barriers. Infection with Agrobacterium tumefaciens is used for transformation of certain plant cells, as described by Shaw et al., Gene, 23:315 (1983) and WO 89/05859 published 29 June 1989. In addition, plants may be transfected using ultrasound treatment as described in WO 91/00358 published 10 January 1991.

For mammalian cells without such cell walls, the calcium phosphate precipitation method of Graham and van der Eb, Virology, 52:456-457 (1978) may be employed. General aspects of mammalian cell host system transformations have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Prokaryotic cells used to produce Apo-2 ligand may be cultured in suitable culture media as described generally in Sambrook et al., supra. Particular forms of culture media that may be employed for culturing E. coli are described further in the Examples below. Mammalian host cells used to produce Apo-2 ligand may be cultured in a variety of culture media.

Examples of commercially available culture media include Ham's F10 (Sigma), Minimal Essential Medium ("MEM", Sigma), RPMI-1640 (Sigma), and Dulbecco's Modified Eagle's Medium ("DMEM", Sigma). Any such media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

In general, principles, protocols, and practical techniques for maximizing the productivity of mammalian cell cultures can be found in Mammalian Cell Biotechnology: A Practical Approach, M. Butler, ed. (IRL Press, 1991).

In accordance with one aspect, one or more divalent metal ions will typically be added to or included in the culture media for culturing or fermenting the host cells. The divalent metal ions are preferably present in or added to the culture media at a concentration level sufficient to enhance storage stability, enhance solubility, or assist in forming stable Apo-2L trimers coordinated by one or more zinc ions. The amount of divalent metal ions which may be added will be dependent, in part, on the host cell density in the culture or potential host cell sensitivity to such divalent metal ions. At higher host cell densities in the culture, it may be beneficial to increase the concentration of divalent metal ions. If the divalent metal ions are added during or after product expression by the host cells, it may be desirable to adjust or increase the divalent metal ion concentration as product expression by the host cells increases. It is generally believed that trace levels of divalent metal ions which may be present in typical commonly available cell culture media may not be sufficient for stable trimer formation. Thus, addition of further quantities of divalent metal ions, as described herein, is preferred.

The divalent metal ions are preferably added to the culture media at a concentration which does not adversely or negatively affect host cell growth, if the divalent metal ions are being added during the growth phase of the host cells in the culture. In shake flask cultures, it was observed that ZnSO₄ added at concentrations of greater than 1 mM can result in lower host cell density. Those skilled in the art appreciate that bacterial cells can sequester metal ions effectively by forming metal ion complexes with cellular matrices. Thus, in the cell cultures, it is preferable to add the selected divalent metal ions to the culture media after the growth phase (after the desired host cell density is achieved) or just prior to product expression by the host cells. To ensure that sufficient amounts of divalent metal ions are present, additional divalent metal ions may be added or fed to the cell culture media during the product expression phase.

The divalent metal ion concentration in the culture media should not exceed the concentration which may be detrimental or toxic to the host cells. In the methods of the invention employing the host cell, *E. coli,* it is preferred that the concentration of the divalent metal ion concentration in the culture media does not exceed about 1mM (preferably, ≤ 1mM). Even more preferably, the divalent metal ion concentration in the culture media is about 50 micromolar to about 250 micromolar. Most preferably, the divalent metal ion used in such methods is zinc sulfate. It is desirable to add the divalent metal ions to the cell culture in an amount wherein the metal ions and Apo-2 ligand trimer can be present at a one to one molar ratio.

The divalent metal ions can be added to the cell culture in any acceptable form. For instance, a solution of the metal ion can be made using water, and the divalent metal ion solution can then be added or fed to the culture media.

Expression of the Apo-2L may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Various labels may be employed, most commonly radioisotopes, and particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionucleotides, fluorescers or enzymes. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected. Gene expression, alternatively, may be measured by immunological methods, such as immunohistochemical staining of cells or tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like.

Antibodies useful for immunohistochemical staining and/or assay of sample fluids may be either monoclonal or polyclonal, and may be prepared in any mammal. Conveniently, the antibodies may be prepared against a native Apo-2 ligand polypeptide or against a synthetic peptide based on the DNA sequences provided herein or against exogenous sequence fused to Apo-2 ligand DNA and encoding a specific antibody epitope.

Apo-2 ligand preferably is recovered from the culture medium as a secreted polypeptide, although it also may be recovered from host cell lysates when directly produced without a secretory signal. If the Apo-2 ligand is membrane-bound, it can be released from the membrane using a suitable detergent solution (e.g. Triton-X 100) or its extracellular region may be released by enzymatic cleavage.

When Apo-2 ligand is produced in a recombinant cell other than one of human origin, the Apo-2 ligand is free of proteins or polypeptides of human origin. However, it is usually necessary to recover or purify Apo-2 ligand from recombinant cell proteins or polypeptides to obtain preparations that are substantially homogeneous as to Apo-2 ligand. As a first step, the culture medium or lysate may be centrifuged to remove particulate cell debris. Apo-2 ligand thereafter is purified from contaminant soluble proteins and polypeptides, with the following procedures being exemplary of suitable purification procedures: by fractionation on an ion-exchange column; ethanol precipitation; reverse phase HPLC; chromatography on silica or on a cation-exchange resin such as DEAE or CM; chromatofocusing; SDS-PAGE; ammonium sulfate precipitation; gel filtration using, for example, Sephadex G-75; diafiltration and protein A Sepharose columns to remove contaminants such as IgG.

In a preferred embodiment, the Apo-2 ligand can be isolated by affinity chromatography. Apo-2 ligand fragments or variants in which residues have been deleted, inserted, or substituted are recovered in the same fashion as native Apo-2 ligand, taking account of any substantial changes in properties occasioned by the variation. For example, preparation of an Apo-2 ligand fusion with another protein or polypeptide, e.g., a bacterial or viral antigen, facilitates purification; an immunoaffinity column containing antibody to the antigen can be used to adsorb the fusion polypeptide.

A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native Apo-2 ligand may require modification to account for changes in the character of Apo-2 ligand or its variants upon expression in recombinant cell culture.

During any such purification steps, it may be desirable to expose the recovered Apo-2L to a divalent metal ion-containing solution or to purification material (such as a chromatography medium or support) containing one or more divalent metal ions. In a preferred embodiment, the divalent metal ions and/or reducing agent is used during recovery or purification of the Apo-2L. Optionally, both divalent metal ions and reducing agent, such as DTT or BME, may be used during recovery or purification of the Apo-2L. It is believed that use of divalent metal ions during recovery or purification will provide for stability of Apo-2L trimer or preserve Apo-2L trimer formed during the cell culturing step.

The description below also relates to methods of producing Apo-2 ligand covalently attached (hereinafter "conjugated") to one or more chemical groups. Chemical groups suitable for use in an Apo-2L conjugate of the present invention are preferably not significantly toxic or immunogenic. The chemical group is optionally selected to produce an Apo-2L conjugate that can be stored and used under conditions suitable for storage. A variety of exemplary chemical groups that can be conjugated to polypeptides are known in the art and include for example carbohydrates, such as those carbohydrates that occur naturally on glycoproteins, polyglutamate, and non-proteinaceous polymers, such as polyols (see, e.g., U.S. Patent No. 6,245,901).

A polyol, for example, can be conjugated to polypeptides such as an Apo-2L at one or more amino acid residues, including lysine residues, as is disclosed in WO 93/00109, supra. The polyol employed can be any water-soluble poly(alkylene oxide) polymer and can have a linear or branched chain. Suitable polyols include those substituted at one or more hydroxyl positions with a chemical group, such as an alkyl group having between one and four carbons. Typically, the polyol is a poly(alkylene glycol), such as poly(ethylene glycol) (PEG), and thus, for ease of description, the remainder of the discussion relates to an exemplary embodiment wherein the polyol employed is PEG and the process of conjugating the polyol to a polypeptide is termed "pegylation." However, those skilled in the art recognize that other polyols, such as, for example, poly(propylene glycol) and polyethylene-polypropylene glycol copolymers, can be employed using the techniques for conjugation described herein for PEG.

The average molecular weight of the PEG employed in the pegylation of the Apo-2L can vary, and typically may range from about 500 to about 30,000 daltons (D). Preferably, the average molecular weight of the PEG is from about 1,000 to about 25,000 D, and more preferably from about 1,000 to about 5,000 D. In one embodiment, pegylation is carried out with PEG having an average molecular weight of about 1,000 D. Optionally, the PEG homopolymer is unsubstituted, but it may also be substituted at one end with an alkyl group. Preferably, the alkyl group is a C1-C4 alkyl group, and most preferably a methyl group. PEG preparations are commercially available, and typically, those PEG preparations suitable for use in the present invention are nonhomogeneous preparations sold according to average molecular weight. For example, commercially available PEG(5000) preparations typically contain molecules that vary slightly in molecular weight, usually ± 500 D.

The Apo-2 ligand used in the invention may be in various forms, such as in monomer form or trimer form (comprising three monomers). Optionally, an Apo-2L trimer will be pegylated in a manner such that a PEG molecule is linked or conjugated to one, two or each of the three monomers that make up the trimeric Apo-2L. In such an embodiment, it is preferred that the PEG employed have an average molecular weight of about 1,000 to about 5,000 D. It is also contemplated that the Apo-2L trimers may be "partially" pegylated, i.e., wherein only one or two of the three monomers that make up the trimer are linked or conjugated to PEG.

A variety of methods for pegylating proteins are known in the art. Specific methods of producing proteins conjugated to PEG include the methods described in U.S. Pat. No. 4,179,337, U.S. Pat. No. 4,935,465 and U.S. Patent No. 5,849,535. Typically the protein is covalently bonded via one or more of the amino acid residues of the protein to a terminal reactive group on the polymer, depending mainly on the reaction conditions, the molecular weight of the polymer, etc. The polymer with the reactive group(s) is designated herein as activated polymer. The reactive group selectively reacts with free amino or other reactive groups on the protein. The PEG polymer can be coupled to the amino or other reactive group on the protein in either a random or a site specific manner. It will be understood, however, that the type and amount of the reactive group chosen, as well as the type of polymer employed, to obtain optimum results, will depend on the particular protein or protein variant employed to avoid having the reactive group react with too many particularly active groups on the protein. As this may not be possible to avoid completely, it is recommended that generally from about 0.1 to 1000 moles, preferably 2 to 200 moles, of activated polymer per mole of protein, depending on protein concentration, is employed. The final amount of activated polymer per mole of protein is a balance to maintain optimum activity, while at the same time optimizing, if possible, the circulatory half-life of the protein.

It is further contemplated that the Apo2L described herein may be also be linked or fused to leucine zipper sequences using techniques known in the art.

Formulations comprising Apo2L/TRAIL and the EGFR inhibitor N-(3-ethynylphenyl)-6,7-bis(2-methoxyethoxy)-4-quinazolinamine, or a pharmaceutically acceptable salt or acid thereof, may be used in accordance with the methods and uses of the present invention. It is believed that such formulations will be particularly suitable for storage as well as for therapeutic administration. The formulations may be prepared by known techniques. For instance, the formulations may be prepared by buffer exchange on a gel filtration column.

Typically, an appropriate amount of a pharmaceutically-acceptable salt is used in the formulation to render the formulation isotonic. Examples of pharmaceutically-acceptable carriers include saline, Ringer's solution and dextrose solution. The pH of the formulation is preferably from about 6 to about 9, and more preferably from about 7 to about 7.5. It will be apparent to those persons skilled in the art that certain carriers may be more preferable depending upon, for instance, the route of administration and concentrations of death receptor agonist and EGFR inhibitor.

Therapeutic compositions can be prepared by mixing the desired molecules having the appropriate degree of purity with optional pharmaceutically acceptable carriers, excipients, or stabilizers (Remington's Pharmaceutical Sciences, 16th edition, Osol, A. ed. (1980)), in the form of lyophilized formulations, aqueous solutions or aqueous suspensions. Acceptable carriers, excipients, or stabilizers are preferably nontoxic to recipients at the dosages and concentrations employed, and include buffers such as Tris, HEPES, PIPES, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; salt-forming counter-ions such as sodium; and/or nonionic surfactants such as TWEEN™, PLURONICS™ or polyethylene glycol (PEG).

Additional examples of such carriers include ion exchangers, alumina, aluminum stearate, lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, colloidal silica, magnesium trisilicate, polyvinyl pyrrolidone, and cellulose-based substances. Carriers for topical or gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations.

Formulations to be used for in vivo administration should be sterile. This is readily accomplished by filtration through sterile filtration membranes, prior to or following lyophilization and reconstitution. The formulation may be stored in lyophilized form or in solution if administered systemically. If in lyophilized form, it is typically formulated in combination with other ingredients for reconstitution with an appropriate diluent at the time for use. An example of a liquid formulation is a sterile, clear, colorless unpreserved solution filled in a single-dose vial for subcutaneous injection.

Therapeutic formulations generally are placed into a container having a sterile access port, for example, an intravenous solution bag or vial having a stopper pierceable by a hypodermic injection needle. The formulations are preferably administered as repeated intravenous (i.v.), subcutaneous (s.c.), intramuscular (i.m.) injections or infusions, or as aerosol formulations suitable for intranasal or intrapulmonary delivery (for intrapulmonary delivery see, e.g., EP 257,956).

The molecules disclosed herein can also be administered in the form of sustained-release preparations. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacrylate) as described by Langer et al., J. Biomed. Mater. Res., 15: 167-277 (1981) and Langer, Chem. Tech., 12: 98-105 (1982) or poly(vinylalcohol)), polylactides (U.S. Patent No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer *et al., supra*), degradable lactic acid-glycolic acid copolymers such as the Lupron Depot (injectable microspheres composed of lactic acid-glycolic acid copolymer and leuprolide acetate), and poly-D-(-)-3-hydroxybutyric acid (EP 133,988). Apo2L/TRAIL and the EGFR inhibitor N-(3-ethynylphenyl)-6,7-bis(2-methoxyethoxy)-4-quinazolinamine, or a pharmaceutically acceptable salt or acid thereof, described herein can be employed in a variety of therapeutic applications. Among these applications are methods of treating various cancers. Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like.

The death receptor agonists and EGFR inhibitors can be administered in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

Effective dosages and schedules for administering the death receptor agonist may be determined empirically, and making such determinations is within the skill in the art. Single or multiple dosages may be employed. It is presently believed that an effective dosage or amount of the death receptor agonist used alone may range from about 1 µg/kg to about 100 mg/kg of body weight or more per day. Interspecies scaling of dosages can be performed in a manner known in the art, e.g., as disclosed in Mordenti et al., Pharmaceut. Res., 8:1351 (1991).

When in vivo administration of the death receptor agonist is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue. Those skilled in the art will understand that the dosage of the death receptor agonist that must be administered will vary depending on, for example, the mammal which will receive the death receptor agonist, the route of administration, and other drugs or therapies being administered to the mammal.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity in Section I above.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the Apo2L variant, or may be given simultaneously therewith.

Sometimes, it may be beneficial to also administer one or more cytokines or growth inhibitory agent.

The death receptor agonists and EGFR inhibitors (and one or more other therapies) may be administered concurrently (simultaneously) or sequentially. In particular embodiments, Apo2L/TRAIL and an EGFR inhibitor are administered concurrently. In another embodiment, Apo2L/TRAIL is administered prior to administration of an EGFR inhibitor. In another embodiment, an EGFR inhibitor is administered prior to Apo2L/TRAIL. Following administration, treated cells *in vitro* can be analyzed. Where there has been in vivo treatment, a treated mammal can be monitored in various ways well known to the skilled practitioner. For instance, tumor cells can be examined pathologically to assay for necrosis or serum can be analyzed for immune system responses.

An article of manufacture such as a kit containing death receptor agonists and EGFR inhibitors useful in the treatment of the disorders described herein comprises at least a container and a label. Suitable containers include, for example, bottles, vials, syringes, and test tubes. The containers may be formed from a variety of materials such as glass or plastic. The label on, or associated with, the container indicates that the formulation is used for treating the condition of choice. The article of manufacture may further comprise a container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a container with another active agent as described above.

The following examples are offered for illustrative purposes only.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### Example 1 - Preparation of compound of formula 4

### Reaction:

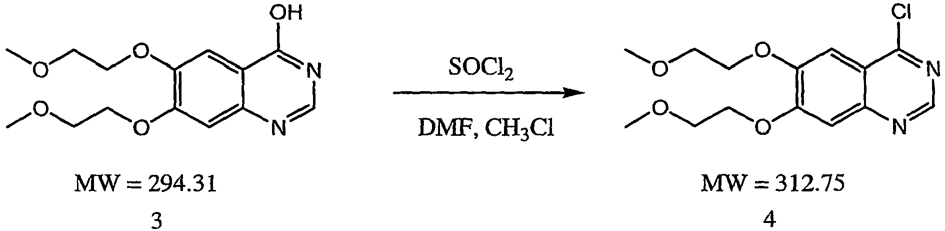

The following materials were used in the synthesis of the compound of formula 4:

| Materials | Quantity | Units | Equivalents/volumes |
|---|---|---|---|
| Compound of formula 3 | 88.0 | kg | 1 equivalent |
| Thionyl chloride | 89.0 | kg | 2.5 equivalents |
| Dimethylformamide | 11 | kg | 0.5 equivalent |
| methylene chloride | 880.0 | L | 10 L/kg |
| 50% NaOH soln | as required | L | 1 equivalent |
| Heptane | 880.0 | L | 10 L/kg |

The following procedure is exemplary of the procedure to follow in the synthesis of the formula 4 compound:
88.0 kg of the compound of formula 3, 880.0 L methylene chloride, and 11.0 kg of dimethylformamide were charged to a clean, dry, glass-lined vessel under nitrogen atmosphere. 89 Kg of thionyl chloride were added to the mix while it is maintained at a temperature of a less than 30°C during the charge. The contents of the reaction vessel were then heated for a minimum of five hours at reflux temperature before sampling for reaction completion and the pH is adjusted to be maintained between 7.0 to 8.0, by using 50% NaOH, as required and the temperature of the reaction mixture is maintained at less than 25°C. The biphasic mixture is stirred for fifteen to twenty minutes and allowed to settle for a minimum of thirty minutes. The layers were separated and the organic layer was concentrated to 1/3 of its volume by removing methylene chloride. 880 L heptane was added with continued distillation of the remaining methylene chloride until the distillate reaches a temperature between 65 and 68°C. The mixture was then cooled to between 10 to 15°C over hours and granulated for a minimum of 1 hour with the solids being isolated by filtration and washed with 220 L heptane. The solids (formula 4 compound) were dried in a vacuum drier at 45 to 50°C.

### Example 2 - Alternative Preparation of Compound of formula 4

In the reaction shown in Example 1, sodium bicarbonate may successfully be used instead of sodium hydroxide as shown in this Example.

| Materials | Quantity | Units | Equivalents/Volumes |
|---|---|---|---|
| Compd 3 | 30.0 | kg | 1 equivalent |
| Thionyl chloride | 36.4 | kg | 3 equivalents |
| Dimethylformamide | 3.75 | kg | 0.5 equivalent |
| methylene chloride | 300 | L | 10 L/kg |
| 50% NaOH soln | as required | L | |
| Heptane | 375 | L | 12.5 L/kg |
| Heptane (wash) | 90 | L | 3 L/kg |
| Sodium Bicarbonate | 64.2 | Kg | 7.5 equivalents |

30.0 kg of the compound of formula 3, 300.0 L methylene chloride, and 3.75 kg of dimethylformamide were charged to a clean, dry, glass-lined vessel under a nitrogen atmosphere.

36.4 kg of thionyl chloride was added to the mix while it was maintained at a temperature of less than 30°C during the charge. The contents of the reaction vessel were then heated at reflux temperature for 13h before sampling for reaction completion. The reaction mixture was cooled to 20-25°C and added slowly to a stirred solution of sodium bicarbonate 64.2 kg and water 274L

cooled to 4°C so that the temperature was maintained at less than 10°C. The final pH of the mixture was adjusted to within the range 7.0 to 8.0 by using 50% sodium hydroxide solution as required. The biphasic mixture was stirred for fifteen to twenty minutes and allowed to settle for a minimum of thirty minutes at 10-20°C. The layers were separated and the organic layer was concentrated to 1/3 of its volume by removing methylene chloride. 375L of heptane was added with continued distillation of the remaining methylene chloride until the distillate reached a temperature between 65 and 68°C. The mixture was then cooled to 0 to 5°C over 4 hours and granulated for a minimum of 1 hour with the solids being isolated by filtration and washed with 90L heptane. The solids (formula 4 compound) were dried in a vacuum drier to 50°C.

### Example 3 -Preparation of compounds 6 and 2 (Step 2)

### Reaction:

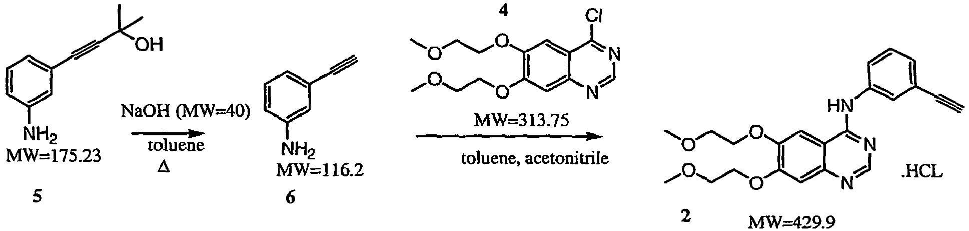

The following materials were used in the synthesis of the compound of formula 6, as intermediate, and the compound of formula 2:

| Materials | Quantity | Units | Equivalents/Volumes |
|---|---|---|---|
| Comp 5 | 61.1 | kg | 1.2 equivalents |
| Toluene | 489 | L | 8 L/kg (WRT to comp 5) |
| NaOH pellets | 4.5 | kg | 0.16 equivalents |
| Filteraid | 0.5 | kg | 0. 0 17 kg/kg (WRT to comp 5) |
| Comp 4 | 90.8 | kg | 1.0 equivalent |
| Acetonitrile | 732 | L | 12 L/kg (WRT to compd 5) |

The following procedure is exemplary of the procedure to follow in the synthesis of the formula 2 compound and intermediate compound of formula 6:
61.1 kg of formula 5 compound, 4.5 kg sodium hydroxide pellets and 489 L toluene were charged to a clean, dry, reaction vessel under nitrogen atmosphere and the reaction temperature is adjusted to between 105 to 108°C. Acetone was removed over four hours by atmospheric distillation while toluene is added to maintain a minimum volume of 6 L of solvent per kg of formula 5 compound. The reaction mixture was then heated at reflux temperature, returning distillates to pot, until the reaction was complete. The mixture was then cooled to between 20 to 25°C, at which time a slurry of 40.0 L toluene and 0.5 kg filteraid was charged to the reaction mixture and the mixture was agitated for ten to fifteen minutes. The resultant material was filtered to remove filteraid, and the cake is washed with 30 L toluene (compound of formula 6).

The filtrate (compound of formula 6) was placed in a clean, dry reaction vessel under nitrogen atmosphere, and 90.8 kg of the compound of formula 4 was charged into the reaction vessel together with 732 L acetonitrile. The reaction vessel was heated to reflux temperature and well agitated. Agitator speed was lowered when heavy solids appear. When the reaction was complete, the contents of reaction vessel were cooled to between to 25°C over three to four hours and the contents were agitated for at least one hour at a temperature between 20 and 25°C. The solids (compound of formula 2, polymorph A form, or mixture of polymorph A and B) were then isolated by filtration and the filter cake was washed with two portions of 50 L acetonitrile and dried under vacuum at a temperature between 40 and 45°C.

It has been discovered that the production of the A polymorph is favored by the reduction of the amount of acetonitrile relative to toluene, and particularly favored if isopropanol is used in place of acetonitrile. However, the use of isopropanol or other alcohols as cosolvents is disfavored because of the propensity to form an ether linkage between the alcoholic oxygen and the 4-carbon of the quinazoline, instead of the desired ethynyl phenyl amino moiety.

It has been further discovered that adjusting the pH of the reaction to between pH 1 and pH 7, particularly between pH 2 and pH 5, for example, between pH 2.5 and pH 4, such as pH 3, will improve the rate of the reaction.

### Example 5 - Recrystallization of compound of formula 2 (which may be in polymorph A form or a mixture of polymorphs A and B) to Polymorph B (Step 3)

### Reaction:

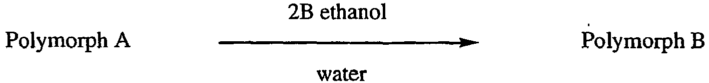

The following materials were used in the conversion of polymorph A (or mixtures of polymorphs A and B) to polymorph B of the compound of formula 2:

| Materials | Quantity | Units | EquivalentsNolumes |
|---|---|---|---|
| Polymorph A (comp 2) | 117.6 | kg | 1 equivalent |
| 2B-ethanol | 1881.6 | L | 16 L/kg |
| Water | 470.4 | L | 4 L/kg |

The following procedure is exemplary of procedures used to convert polymorph A (or mixtures of polymorphs A and B) into the more thermodynamically stable polymorph B of the compound of formula 2:
117.6 kg of the polymorph A (or mixtures of polymorphs A and B) were charged to a clean, dry, reaction vessel together 1881.6 L 2B-ethanol and 470.4 L water under a nitrogen atmosphere. The temperature was adjusted to reflux (-80°C) and the mixture was agitated until the solids dissolve. The solution was cooled to between 65 and 70°C and clarified by filtration. With low speed agitation, the solution was further cooled to between 50 and 60°C over a minimum time of 2 hours and the precipitate was granulated for 2 hours at this temperature. The mixture was further cooled to between 0 and 5°C over a minimum time of 4 hours and granulated for a minimum of 2 hours at this temperature. The solids (polymorph B) were isolated by filtration and washed with at least 100 L 2B-ethanol. The solids were determined to be crystalline polymorph B form of [6,7-bis(2-methoxyethoxy)quinazolin-4-yl]-(3-ethynyiphenyl)-amine hydrochloride substantially free of the polymorph A form. The solids obtained by this method are substantially homogeneous polymorph B form crystals relative to the polymorph A form. The method allows for production of polymorph B in an amount at least 70% by weight, at least 80% by weight, at least 90% by weight, at least 95% by weight, and at least 98% by weight relative to the weight of the polymorph A. It is to be understood that the methods described herein are only exemplary and are not intended to exclude variations in the above parameters which allow the production of polymorph B in varying granulations and yields, according to the desired storage, handling and manufacturing applications of the compound. The solids were vacuum dried at a temperature below 50°C and the resultant product was milled to provide the polymorph B in usable form.

Polymorph B exhibits an X-ray powder diffraction pattern having characteristic peaks expressed in degrees 2-theta at approximately 6.26, 12.48, 13.39, 16.96, 20.20, 21.10, 22.98, 24.46, 25.14 and 26.91.

### Example 5 -Analysis of Apo2L/TRAIL receptor and EGFR expression in H460 cell lines

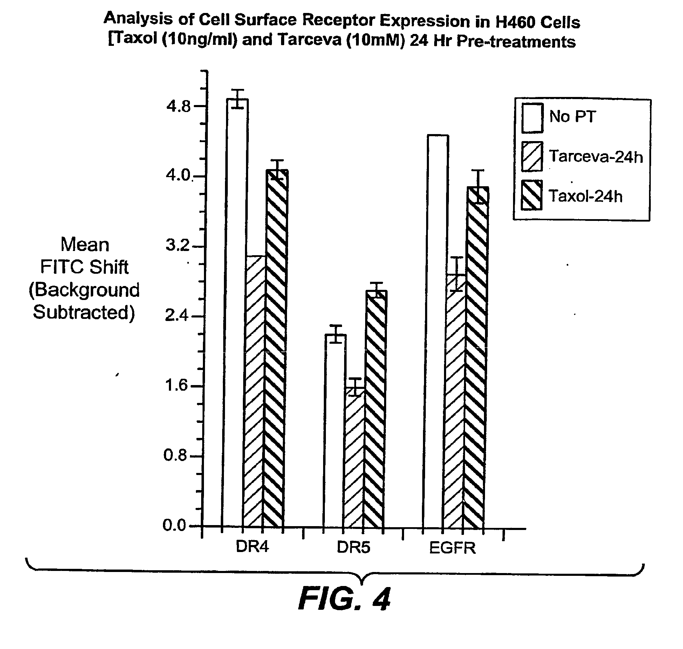

To examine the cell-surface expression of Apo2L/TRAIL receptors (DR4, DR5) and EGFR in human non-small lung cancer cell lines, the H460 cell line (ATCC) was analyzed by FACS using monoclonal antibodies specific for DR4 (mAb 4H6.17.8; ATCC HB-12455), DR5 (mAb 3H3.14.5; HB-12534), and EGFR. The cells were either pretreated with Tarceva™, Taxol^{®}, or alternatively received no pretreatment, as indicated in Figure 4. The bar diagram in Figure 4 illustrates the respective receptor expression levels.

### Example 6 - Effects of Apo2L/TRAIL, Tarceva™, or combination treatment on the growth of cancer cells in vitro

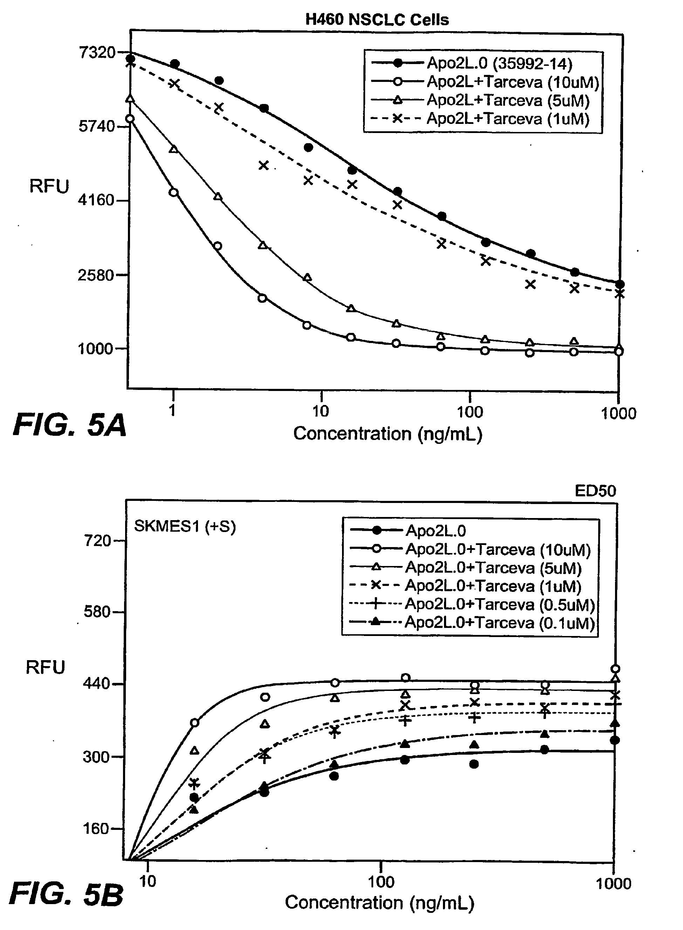

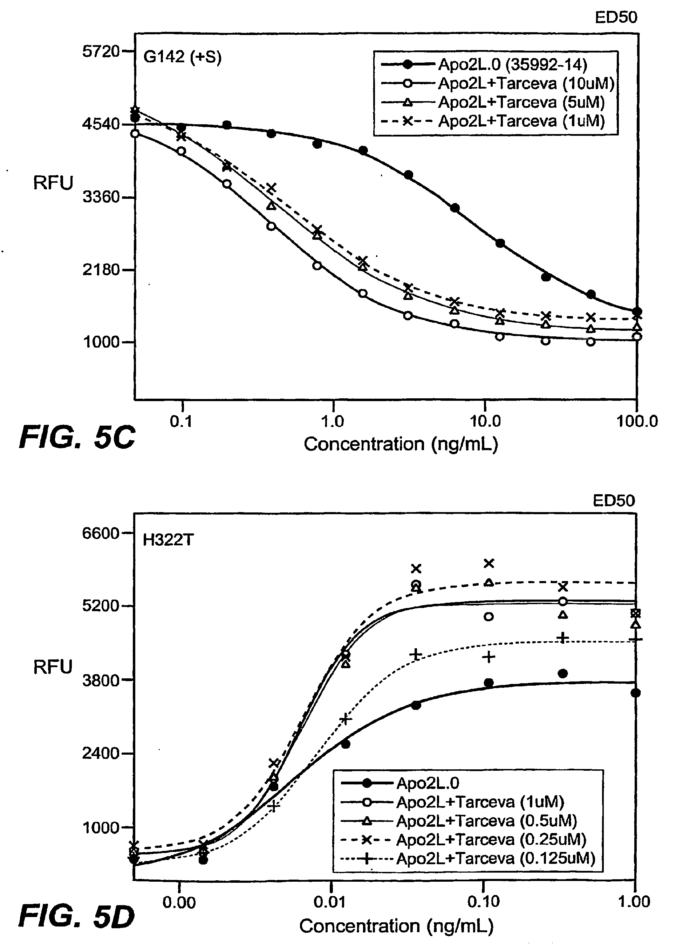

Serial dilutions of H460, SKMES1, G142, and H332T cells were performed in 96-well tissue culture plates (Falcon). The effects of Apo-2 ligand (amino acids 114-281, described in PCT US00/17579) and Tarceva™ were tested, as described in Figures 5A-5D. The plates were incubated at 37°C for 24 hours. AlamarBlue (Trek Diagnostic Systems, Inc.) was added to the wells for the last 3 hours of the 24 hours incubation time. Fluorescence was read using a 96-well fluorometer with excitation at 530 nm and emission of 590 nm. The results are expressed in relative fluorescence units (RFU). For data analysis the 4-parameter curve fitting program (Kaleidagraph) was used.

The results of the bioassays are shown in Figs. 5A-5D.

### Example 7- Effect of Apo2L/TRAIL, Tarceva™, or combination treatment on the growth of H460 tumor xenografts in vivo

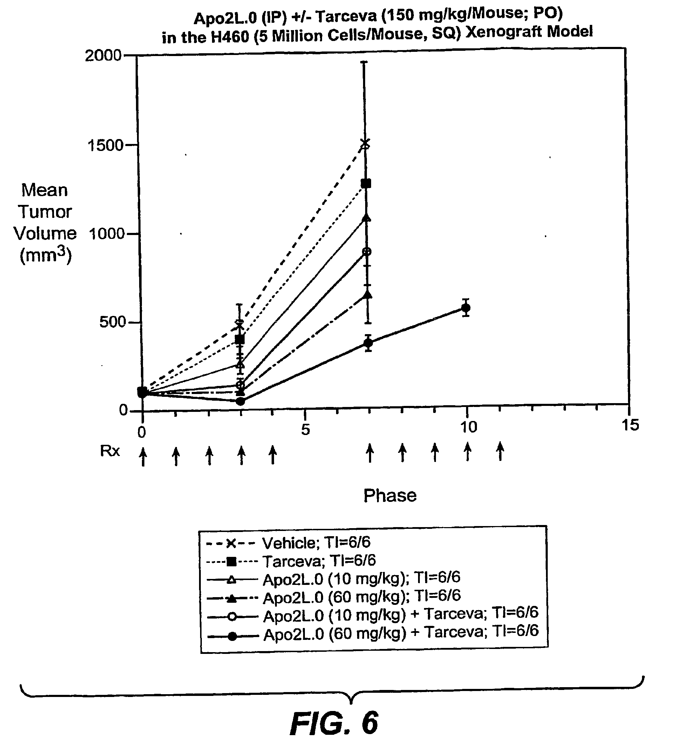

Mice were injected subcutaneously with H460 non-small lung cancer cells (ATCC) (5 million cells per mouse). The mice were then divided into 6 study groups (6 mice per group) and treated with vehicle, Tarceva™ (Genentech, Inc.), Apo2L.0 (Apo2L/TRAIL polypeptide consisting of amino acids 114-281 of Fig. 1 (see Ashkenazi et al., J. Clin. Invest., 104:155-162 (1999)) or combinations thereof, as provided in Figure 6. Tumors in vehicle-treated mice grew rapidly, while Apo2L/TRAIL and Tarceva™ treatment markedly delayed tumor growth.

## Claims

1. Apo2L/TRAIL polypeptide and an EGFR inhibitor for use in a method for treating cancer by enhancing apoptosis in one or more mammalian cells, wherein said EGFR inhibitor is N-(3-ethynylphenyl)-6,7-bis(2-methoxyethoxy)-4-quinazolinamine, or a pharmaceutically acceptable salt or acid thereof.

2. Use of Apo2L/TRAIL polypeptide and EGFR inhibitor in the preparation of a medicament for treating cancer by enhancing apoptosis in one or more mammalian cells, wherein said EGFR inhibitor is N-(3-ethynylphenyl)-6,7-bis(2-methoxyethoxy)-4-quinazolinamine, or a pharmaceutically acceptable salt or acid thereof.

3. An *in vitro* method of enhancing apoptosis in one or more mammalian cells comprising exposing said cells to an effective amount of Apo2L/TRAIL polypeptide and EGFR inhibitor, wherein said EGFR inhibitor is N-(3-ethynylphenyl)-6,7-bis(2-methoxyethoxy)-4-quinazolinamine, or a pharmaceutically acceptable salt or acid thereof.

4. The composition for use in a method for treatment, the use or the method of any one of claims 1 to 3, wherein said cells are exposed to the EGFR inhibitor prior to being exposed to the Apo2L/TRAIL polypeptide.

5. The composition for use in a method for treatment, the use or the method of any one of claims 1 to 3, wherein said cells are exposed to the Apo2L/TRAIL polypeptide prior to being exposed to the EGFR inhibitor.

6. The composition for use in a method for treatment, the use or the method of any one of claims 1 to 3, wherein said cells are exposed simultaneously to the EGFR inhibitor and the Apo2L/TRAIL polypeptide.

7. The composition for use in a method for treatment, the use or the method of any one claims 1 to 3, wherein said Apo2L/TRAIL is a fragment of the polypeptide of SEQ ID NO: 1.

8. The composition for use in a method for treatment, the use or the method of claim 7, wherein said Apo2L/TRAIL fragment comprises the extracellular domain of the polypeptide of SEQ ID NO: 1.

9. The composition for use in a method for treatment, the use or the method of any one of claims 1 to 3, wherein the the Apo2L/TRAIL polypeptide is an Apo2L/TRAIL polypeptide variant having at least about 90% amino acid sequence identity with the extracellular domain of SEQ ID NO: 1.

10. The composition for use in a method for treatment, the use or the method of claim 9, wherein said Apo2L/TRAIL variant has at least about 95% amino acid sequence identity with the extracellular domain of SEQ ID NO: 1.

11. The composition for use in a method for treatment, the use or the method of claim 7, wherein said fragment comprises amino acids 114-281 of SEQ ID NO: 1.

12. The composition for use in a method for treatment, the use or the method of claim 11, wherein the Apo2L/TRAIL fragment comprising amino acids 114-281 of SEQ ID NO:1 is linked to one or more polyethylene glycol (PEG) molecules.

13. The composition for use in a method for treatment, the use or the method of any one of the preceding claims, wherein said cancer is selected from the group consisting of small-cell lung cancer, non-small cell lung cancer, colon cancer, colorectal cancer, and pancreatic cancer.

14. The composition for use in a method for treatment, the use or the method of any one of the preceding claims, wherein said cancer is colon cancer, colorectal cancer, small-cell lung cancer or non-small cell lung cancer.

15. The composition for use in a method for treatment, the use or the method of any one of claims 1 to 14 further comprising exposing the cancer cells to one or more growth inhibitory agents.

16. The composition for use in a method for treatment, the use or the method of any one of claims 1 to 15 further comprising exposing the cells to radiation.

## Patentansprüche

1. Apo2L/TRAIL-Polypeptid und ein EGFR-Hemmer zur Verwendung in einem Verfahren zur Behandlung von Krebs durch die Steigerung von Apoptose in einer oder mehreren Säugetierzellen, worin der EGFR-Hemmer N-(3-Ethinylphenyl)-6,7-bis(2-methoxyethoxy)-4-chinazolinamin oder ein pharmazeutisch annehmbares Salz davon ist.

2. Verwendung eines Apo2L/TRAIL-Polypeptids und eines EGFR-Hemmers zur Herstellung eines Medikaments zur Behandlung von Krebs durch die Steigerung von Apoptose in einer oder mehreren Säugetierzellen, worin der EGFR-Hemmer N-(3-Ethinylphenyl)-6,7-bis(2-methoxyethoxy)-4-chinazolinamin oder ein pharmazeutisch annehmbares Salz davon ist.

3. In-vitro-Verfahren zur Steigerung von Apoptose in einer oder mehreren Säu-getierzellen, das umfasst, die Zellen einer wirksamen Menge eines Apo2L/TRAIL-Polypeptids und eines EGFR-Hemmers auszusetzen, worin der EGFR-Hemmer N-(3-Ethinylphenyl)-6,7-bis(2-methoxyethoxy)-4-chinazolinamin oder ein pharmazeutisch annehmbares Salz davon ist.

4. Zusammensetzung zur Verwendung in einem Behandlungsverfahren, Verwendung oder Verfahren nach einem der Ansprüche 1 bis 3 worin die Zellen dem EGFR-Hemmer ausgesetzt werden, bevor sie dem Apo2L/TRAIL-Polypeptid ausgesetzt werden.

5. Zusammensetzung zur Verwendung in einem Behandlungsverfahren, Verwendung oder Verfahren nach einem der Ansprüche 1 bis 3, worin die Zellen dem Apo2L/TRAIL-Polypeptid ausgesetzt werden, bevor sie dem EGFR-Hemmer ausgesetzt werden.

6. Zusammensetzung zur Verwendung in einem Behandlungsverfahren, Verwendung oder Verfahren nach einem der Ansprüche 1 bis 3, worin die Zellen dem Apo2L/TRAIL-Polypeptid und dem EGFR-Hemmer gleichzeitig ausgesetzt werden.

7. Zusammensetzung zur Verwendung in einem Behandlungsverfahren, Verwendung oder Verfahren nach einem der Ansprüche 1 bis 3, worin das Apo2L/TRAIL-Polypeptid ein Fragment des Polypeptids von Seq.-ID Nr. 1 ist.

8. Zusammensetzung zur Verwendung in einem Behandlungsverfahren, Verwendung oder Verfahren nach Anspruch 7, worin das Apo2L/TRAIL-Fragment die extra-zelluläre Domäne des Polypeptids von Seq.-ID Nr. 1 umfasst.

9. Zusammensetzung zur Verwendung in einem Behandlungsverfahren, Verwendung oder Verfahren nach einem der Ansprüche 1 bis 3, worin das Apo2L/TRAIL-Polypeptid eine Apo2L/TRAIL-Polypeptidvariante ist, die zumindest etwa 90 % Aminosäuresequenzidentität in Bezug auf die extrazelluläre Domäne von Seq.-ID Nr. 1 aufweist.

10. Zusammensetzung zur Verwendung in einem Behandlungsverfahren, Verwen-dung oder Verfahren nach Anspruch 9, worin die Apo2L/TRAIL-Variante zumindest etwa 95 % Aminosäuresequenzidentität in Bezug auf die extrazelluläre Domäne von Seq.-ID Nr. 1 aufweist.

11. Zusammensetzung zur Verwendung in einem Behandlungsverfahren, Verwendung oder Verfahren nach Anspruch 7, worin das Fragment die Aminosäuren 114 bis 281 von Seq.-ID Nr. 1 umfasst.

12. Zusammensetzung zur Verwendung in einem Behandlungsverfahren, Verwendung oder Verfahren nach Anspruch 11, worin das Apo2L/TRAIL-Fragment, das die Aminosäuren 114 bis 281 von Seq.-ID Nr. 1 umfasst, an ein oder mehrere Polyethy-lenglykol- (PEG-) Moleküle gebunden ist.

13. Zusammensetzung zur Verwendung in einem Behandlungsverfahren, Verwendung oder Verfahren nach einem der vorangegangenen Ansprüche, worin der Krebs aus der aus kleinzelligem Lungenkrebs, nicht kleinzelligem Lungenkrebs, Kolonkrebs, Kolorektalkrebs und Pankreaskrebs bestehenden Gruppe ausgewählt ist.

14. Zusammensetzung zur Verwendung in einem Behandlungsverfahren, Verwendung oder Verfahren nach einem der vorangegangenen Ansprüche, worin der Krebs Kolonkrebs, Kolorektalkrebs, kleinzelliger Lungenkrebs oder nicht kleinzelliger Lungenkrebs ist.

15. Zusammensetzung zur Verwendung in einem Behandlungsverfahren, Verwendung oder Verfahren nach einem der Ansprüche 1 bis 14, die/das weiters das Aussetzen der Krebszellen gegenüber einem oder mehreren wachstumshemmenden Mitteln umfasst.

16. Zusammensetzung zur Verwendung in einem Behandlungsverfahren, Verwendung oder Verfahren nach einem der Ansprüche 1 bis 15, die/das weiters das Aussetzen der Krebszellen gegenüber Strahlung umfasst.

## Revendications

1. Polypeptide Apo2L/TRAIL et inhibiteur d'EGFR pour une utilisation dans une méthode de traitement du cancer par amplification de l'apoptose dans une ou plusieurs cellules de mammifère, ledit inhibiteur d'EGFR étant la N-(3-éthynylphényl)-6,7-bis(2-méthoxyéthoxy)-4-quinazolinamine ou l'un de ses sels pharmaceutiquement acceptables ou acides.

2. Utilisation de polypeptide Apo2L/TRAIL et d'inhibiteur d'EGFR dans la préparation d'un médicament destiné au traitement du cancer par amplification de l'apoptose dans une ou plusieurs cellules de mammifère, ledit inhibiteur d'EGFR étant la N-(3-éthynylphényl)-6,7-bis(2-méthoxyéthoxy)-4-quinazolinamine ou l'un de ses sels pharmaceutiquement acceptables ou acides.

3. Méthode *in vitro* d'amplification de l'apoptose dans une ou plusieurs cellules de mammifère comprenant l'exposition desdites cellules à une quantité efficace de polypeptide Apo2L/TRAIL et d'inhibiteur d'EGFR, ledit inhibiteur d'EGFR étant la N-(3-éthynylphényl)-6,7-bis(2-méthoxyéthoxy)-4-quinazolinamine ou l'un de ses sels pharmaceutiquement acceptables ou acides.

4. Composition pour une utilisation dans une méthode de traitement, utilisation ou méthode selon l'une quelconque des revendications 1 à 3, où lesdites cellules sont exposées à l'inhibiteur d'EGFR avant d'être exposées au polypeptide Apo2L/TRAIL.

5. Composition pour une utilisation dans une méthode de traitement, utilisation ou méthode selon l'une quelconque des revendications 1 à 3, où lesdites cellules sont exposées au polypeptide Apo2L/TRAIL avant d'être exposées à l'inhibiteur d'EGFR.

6. Composition pour une utilisation dans une méthode de traitement, utilisation ou méthode selon l'une quelconque des revendications 1 à 3, où lesdites cellules sont exposées simultanément à l'inhibiteur d'EGFR et au polypeptide Apo2L/TRAIL.

7. Composition pour une utilisation dans une méthode de traitement, utilisation ou méthode selon l'une quelconque des revendications 1 à 3, où ledit Apo2L/TRAIL est un fragment du polypeptide de SEQ ID NO : 1.

8. Composition pour une utilisation dans une méthode de traitement, utilisation ou méthode selon la revendication 7, où ledit fragment de Apo2L/TRAIL comprend le domaine extracellulaire du polypeptide de SEQ ID NO : 1.

9. Composition pour une utilisation dans une méthode de traitement, utilisation ou méthode selon l'une quelconque des revendications 1 à 3, où le polypeptide Apo2L/TRAIL est un variant du polypeptide Apo2L/TRAIL présentant au moins environ 90 % d'identité de séquence d'acides aminés avec le domaine extracellulaire de SEQ ID NO : 1.

10. Composition pour une utilisation dans une méthode de traitement, utilisation ou méthode selon la revendication 9, où ledit variant de Apo2L/TRAIL présente au moins environ 95 % d'identité de séquence d'acides aminés avec le domaine extracellulaire de SEQ ID NO : 1.

11. Composition pour une utilisation dans une méthode de traitement, utilisation ou méthode selon la revendication 7, où ledit fragment comprend les acides aminés 114 à 281 de SEQ ID NO : 1.

12. Composition pour une utilisation dans une méthode de traitement, utilisation ou méthode selon la revendication 11, où le fragment de Apo2L/TRAIL comprenant les acides aminés 114 à 281 de SEQ ID NO : 1 est lié à une ou plusieurs molécules de polyéthylèneglycol (PEG).

13. Composition pour une utilisation dans une méthode de traitement, utilisation ou méthode selon l'une quelconque des revendications précédentes, où ledit cancer est choisi dans le groupe consistant en un cancer du poumon à petites cellules, un cancer du poumon non à petites cellules, un cancer du côlon, un cancer colorectal et un cancer pancréatique.

14. Composition pour une utilisation dans une méthode de traitement, utilisation ou méthode selon l'une quelconque des revendications précédentes, où ledit cancer est un cancer du côlon, un cancer colorectal, un cancer du poumon à petites cellules ou un cancer du poumon non à petites cellules.

15. Composition pour une utilisation dans une méthode de traitement, utilisation ou méthode selon l'une quelconque des revendications 1 à 14, comprenant en outre l'exposition des cellules cancéreuses à un ou plusieurs agents inhibiteurs de la croissance.

16. Composition pour une utilisation dans une méthode de traitement, utilisation ou méthode selon l'une quelconque des revendications 1 à 15, comprenant en outre l'exposition des cellules à des radiations.
